# EUROPÄISCHE PATENTSCHRIFT

(11) **EP 1 816 989 B1**
(45) Veröffentlichungstag und Bekanntmachung des Hinweises auf die Patenterteilung: **02.02.2011**
(21) Anmeldenummer: 05807161.4
(22) Anmeldetag: 18.10.2005
(51) Int. Cl.: A61F 2/44

(54) **BEWEGUNGSPHYSIOLOGISCHE BANDSCHEIBENENDOPROTHESE FÜR DIE LENDEN- UND HALSWIRBELSÄULE**
PHYSIOLOGICAL INTERVERTEBRAL DISK ENDOPROSTHESIS FOR THE LUMBAR COLUMN AND CERVICAL VERTEBRAL COLUMN
PROTHESE DE DISQUE INTERVERTEBRAL PHYSIOLOGIQUE DESTINEE AUX LOMBAIRES ET AUX CERVICALES

(30) Priorität: 18.10.2004 WO PCT/DE2004/002331
(43) Veröffentlichungstag der Anmeldung: 15.08.2007
(73) Patentinhaber: Büttner-Janz, Karin, 12557 Berlin (DE)
(72) Erfinder: BÜTTNER, Eiko, 10245 Berlin (DE)
(74) Vertreter: Harrison, Robert John
(86) Internationale Anmeldenummer: PCT/DE2005/001884
(87) Internationale Veröffentlichungsnummer: WO 2006/042532

(56) Entgegenhaltungen:
- EP-A- 0 560 141
- WO-A-00/53127
- WO-A-2004/064692
- US-A- 5 258 031

## Beschreibung

Die Erfindung betrifft eine Bandscheibenendoprothese für den vollständigen Ersatz der Bandscheibe im Lenden- und Halswirbelsäulenbereich.

Die Idee des funktionserhaltenden künstlichen Bandscheibenersatzes ist zwar jünger als der endoprothetische Ersatz der Extremitätengelenke, jedoch inzwischen fast 50 Jahre alt [Büttner-Janz, Hochschuler, McAfee (Eds.): The Artificial Disc. Springer Verlag, Berlin, Heidelberg, New York 2003] Sie resultiert aus biomechanischen Überlegungen, nicht zufrieden stellenden Ergebnissen von Versteifungsoperationen, Erkrankungen in der Nachbarschaft von Versteifungen und aus der Entwicklung neuer Materialien mit Langzeitbeständigkeit.

Mithilfe eines funktionserhaltenden Bandscheibenimplantats ist es möglich, eine Versteifungsoperation zu umgehen, d.h. die Bewegung im Zwischenwirbelraum zu erhalten bzw. wieder herzustellen. Durch die Implantation einer künstlichen Bandscheibe gelingt es auch, im in-vitro-Experiment die biomechanischen Eigenschaften des Bewegungssegments nach einer Nukleotomie weitgehend zu normalisieren.

Unterschieden werden Implantate zum Ersatz der gesamten Bandscheibe von solchen zum Ersatz des Nucleus pulposus. Implantate zum kompletten Bandscheibenersatz sind entsprechend voluminös; sie werden von ventral oder ventrolateral eingebracht. Eine Implantation im unmittelbaren Anschluss an eine standardmäßige Nukleotomie kann mit einer Prothese zum kompletten Bandscheibenersatz demzufolge nicht durchgeführt werden.

Die Indikation zum funktionserhaltenden Bandscheibenersatz umfasst, als Alternative zur operativen Fusion, neben der primären schmerzhaften Diskopathie auch voroperierte Patienten mit einem sogenannten Postdiskotomiesyndrom, Patienten, die einen wiederholten Bandscheibenvorfall in der gleichen Etage aufweisen und Patienten, die nach einer Versteifungsoperation eine Anschlusssymptomatik in einer Nachbarbandscheibe haben.

Insgesamt werden derzeit über 10 verschiedene Prothesen zum totalen Bandscheibenersatz klinisch eingesetzt. Besonders bekannt sind bei der Lendenwirbelsäule die Charité Artificial Disc, die Prodisc, die Maverick, die FlexiCore und die Mobidisc (Übersicht in Clinica Reports, PJB Publications Ltd., Juni 2004) und bei der Halswirbelsäule die Bryan Prothese, die Prestige LP Prothese, die Prodisc-C und die PCM Prothese, welche im Folgenden beschrieben werden.

Die Prodisc Prothese für die Lendenwirbelsäule wird seit einer Weiterentwicklung zur Prodisc II seit 1999 implantiert. Es ist eine nach den Komponenten zwar 3-teilige, jedoch funktionell 2-teilige Bandscheibenprothese in der Gleitpaarung Metall-Polyethylen. Implantationen mit der Prodisc werden in der Lendenwirbelsäule und mit einem adaptierten Prothesenmodell, der Prodisc-C, auch in der Halswirbelsäule durchgeführt. Es stehen unterschiedliche Größen, Höhen (über den Polyethylenkern) und Lordosewinkel (über die Metall-Abschlussplatten) zur Verfügung. Das Vor- und Rückneigen sowie Rechts- und Linksneigen sind bei der Prothese in einem gleich großen Bewegungsumfang möglich, die Axialrotation wird konstruktionsgemäß nicht begrenzt.

Gleiches trifft zu für die beiden 2-teiligen Prothesen der Halswirbelsäule, die PCM Prothese in der Gleitpaarung Metall-Polyethylen und die Prestige LP Prothese in der Gleitpaarung Metall-Metall. Als Besonderheit weist die Prestige LP Prothese konstruktionsgemäß die Möglichkeit einer anterior-posterioren Translation auf, infolge der horizontal nach ventral verlängerten Konkavität, die im Frontalschnitt den gleichen Radius hat wie die Konvexität.

Die Maverick und die FlexiCore für die Lendenwirbelsäule sind funktionell 2-teilige Prothesen mit sphärischen konvex-konkaven Gleitpartnern, beide in einer Metall-Metall-Gleitpaarung. Die Mobidisc ist dagegen eine funktionell 3-teilige Prothese in der Gleitpaarung Metall-Polyethylen mit 2 Artikulationsbereichen. Der eine Bereich ist wie bei den vorgenannten 3 Prothesen ein Ausschnitt einer Kugel mit je einer konvexen und einer konkaven Fläche der artikulierenden Partner von gleichem Radius und der andere Bereich der Mobidisc ist plan. Obwohl im planen Bereich eine Abbremsung der Axialrotation vorgesehen ist, ist diese jedoch im konvex-konkaven Artikulationsbereich nicht limitiert. Dagegen weist die FlexiCore innerhalb der sphärischen Gleitflächen über einen schmalen Bereich eines Anschlags eine Rotationsbegrenzung auf.

Als kompakte Prothese für den kompletten Bandscheibenersatz der Halswirbelsäule ist die Bryan Prothese im klinischen Einsatz, die über konvexe Titanplatten mit poröser Oberfläche an den Wirbelkörpern fixiert ist und ihre biomechanischen Eigenschaften aus einem Polyurethan-Nucleus erhält.

Die längsten Erfahrungen mit totalem Bandscheibenersatz liegen mit der Charité Prothese vor, welche Gegenstand der DE 35 29 761 C2 und der US 5,401,269 ist. Diese Prothese wurde im Jahr 1982 von Dr. Schellnack und Dr. Büttner-Janz an der Berliner Charité entwickelt und später als SB Charité Prothese benannt. 1984 erfolgte die erste Operation. Die Bandscheibenprothese wurde weiterentwickelt und seit 1987 wird der aktuelle Typ dieser Prothese, Modell III, implantiert; inzwischen weltweit über 10000mal (DE 35 29 761 C2, US 5,401,269). Die Prothese ist funktionell 3-teilig in der Gleitpaarung Metall-Polyethylen in 2 gleichen sphärischen Gleitflächen, die zum einen der transversal sich bewegende Polyethylenkern aufweist, und zum anderen die entsprechend adaptierten konkaven Pfannen in den beiden Metall-Abschlussplatten. Für die Anpassung an die Anatomie des Zwischenwirbelraums stehen in der Fläche unterschiedliche Größen der Metallplatten der Charité Prothese und verschiedene Höhen der größenadaptierten Gleitkerne sowie winklige Prothesen-Abschlussplatten zur Verfügung, die, in sagittaler Richtung umgekehrt implantiert, auch als Wirbelkörperersatz dienen können. Die Primärverankerung der Charité Prothese erfolgt über 6 Zähnchen, die sich zu dritt leicht zur Mitte versetzt neben dem vorderen und hinteren konvexen Rand jeder Prothesenplatte befinden.

Die anderen Prothesen weisen bei den wirbelkörperseitigen Metallplatten andere Primärverankerungen auf, z.B. einen Kiel, der sagittal verläuft, eine strukturierte Oberfläche, eine konvexe Form mit z.B. quer verlaufenden Rillen und Kombinationen davon, auch mit unterschiedlich lokalisierten Zähnchen. Darüber hinaus können Verschraubungen zur Anwendung kommen, entweder von ventral oder von intern im Zwischenwirbelraum in den Wirbelkörper hinein.

Um die Verankerung der Prothesen-Abschlussplatten an den Wirbelkörpern langfristig zusätzlich zu gewährleisten und somit eine feste Verbindung mit dem Knochen zu erzeugen, wurde analog zu zementfreien Hüft- und Knieprothesen eine Oberfläche geschaffen, die Chrom-Kobalt, Titan und Kalziumphosphat so miteinander verbindet, dass Knochen direkt an die Abschlussplatten heranwachsen kann. Diese Verbindung zwischen Prothese und Knochen, ohne Ausbildung von Bindegewebe, ermöglicht eine lang andauernde Fixierung der künstlichen Bandscheibe und reduziert die Gefahr von Prothesenlockerungen, Verschiebungen der Prothese und Materialbrüchen.

Ein Hauptziel beim funktionserhaltenden Bandscheibenersatz besteht darin, die Bewegungsabläufe der Prothese weitgehend dem Bewegungsmuster einer gesunden Bandscheibe anzupassen. Im unmittelbaren Zusammenhang damit steht die Bewegung und Belastung in den Wirbelbogengelenken, die bei einer Fehlbeanspruchung ein eigenes Krankheitspotential haben. Es kann zu einer Abnutzung der Wirbelbogengelenke kommen (Arthrose, Spondylarthrose), im Vollbild mit der Ausbildung von Osteophyten. Durch diese Osteophyten und auch bei einem pathologischen Bewegungsmuster der Bandscheibe allein, ist die Reizung von Nervenstrukturen möglich.

Die gesunde Bandscheibe ist im Zusammenwirken mit den anderen Elementen des Bewegungssegments so aufgebaut, dass nur bestimmte Bewegungsumfänge möglich sind. So werden in der Bandscheibe zum Beispiel Vor- und Rückwärtsbewegungen des Rumpfes mit Drehbewegungen verbunden und auch Seitbewegungen kombiniert mit anderen Bewegungen ausgeführt. Die Bewegungsausschläge sind, bezogen auf die Extension (Rückneigen) und Flexion (Vorneigen) sowie das Seitneigen nach rechts und links und auch bezogen auf die Rotation, bei einer gesunden Bandscheibe im Ausmaß sehr unterschiedlich. Trotz übereinstimmender Grundmerkmale bestehen darüber hinaus Unterschiede in den Bewegungsausschlägen zwischen der Lenden- und Halswirbelsäule.

Bei Bewegungen in der Bandscheibe kommt es zu Veränderungen des Drehzentrums, d.h., die Bewegungen in der Bandscheibe erfolgen nicht um ein fixiertes Zentrum, sondern infolge einer simultanen Translationsbewegung der benachbarten Wirbel verändert das Zentrum stetig seine Lage (inkonstantes Rotationszentrum). Die Prothese nach der DE 35 29 761 C2 zeigt hierzu einen Aufbau, der sie von anderen verfügbaren Prothesentypen, welche wie ein Kugelgelenk aufgebaut sind und sich demzufolge nur um einen definiert lokalisierten Drehpunkt bewegen, unterscheidet. Durch den dreiteiligen Aufbau der Prothese nach der DE 35 29 761 C2 aus zwei metallischen Abschlussplatten und dem dazwischen liegenden, frei beweglichen Gleitkern aus Polyethylen wird der Bewegungsablauf der gesunden Bandscheibe in der humanen Wirbelsäule weitgehend nachempfunden, ausgenommen jedoch die exakten Bewegungsausschläge in die einzelnen Bewegungsrichtungen.

Ein weiteres wichtiges Merkmal der gesunden lumbalen Bandscheiben ist deren Trapezform, die für die Lordose der Lenden- und Halswirbelsäule hauptverantwortlich ist. Die Wirbelkörper selbst sind an der Lordose nur in geringem Ausmaß beteiligt. Bei einem endoprothetischen Ersatz der Bandscheiben sollte die Lordose möglichst erhalten bleiben bzw. rekonstruiert werden. Bei der Charité Bandscheibenprothese gibt es dafür vier verschieden gewinkelte Abschlussplatten, die zudem untereinander kombiniert werden können. Jedoch bedeutet es intraoperativ einen gewissen Aufwand und die Gefahr einer Schädigung der Wirbelkörperendplatten mit erhöhter Gefahr für ein Einsinken der Prothese in die Wirbelkörper, wenn nach der Implantation der Prothese diese komplett wieder entnommen werden muss, weil eine gute Lordoseeinstellung und Belastung des Polyethylenkernzentrums nicht erzielt werden konnten.

Um ein Abgleiten bzw. Herausrutschen des mittleren Gleitpartners aus den beiden Abschlussplatten zu verhindern, ist aus der DE 35 29 761 C2 ein Gleitkern mit einer zweiseitigen teilsphärischen Oberfläche (linsenförmig), mit einem planen Führungsrand und außen mit einer Ringwulst versehen, bekannt, der sich bei Extrembewegungen zwischen den beiden formadaptierten Abschlussplatten verklemmt. Auch aus der DE 102 42 329 A1 ist eine ähnliche Bandscheibenprothese bekannt, die um die Kontaktflächen herum eine Rille aufweist, in der ein mit der gegenüberliegenden Kontaktfläche in Kontakt befindlicher elastischer erster Ring zur besseren Führung eingebettet ist.

Die EP 0 560 141 B1 beschreibt eine 3-teilige Bandscheibenendoprothese, welche ebenfalls aus zwei Abschlussplatten und einem dazwischen lokalisierten Prothesenkern besteht. Die in dieser Druckschrift beschriebene Bandscheibenendoprothese setzt bei Drehung ihrer Abschlussplatten in entgegen gesetzte Richtungen um eine vertikale Hochachse der Rotation ohne Anschläge an den Prothesenplatten einen Widerstand entgegen. Dies wird durch ein Aufgleiten der Abschlussplatten bei der Rotation auf den Prothesenkern durch das Gewicht, welches auf die Platten infolge der biomechanischen Lastübertragung in der Wirbelsäule einwirkt, erreicht, da sich im mittigen Sagittal- und Frontalschnitt die jeweiligen Krümmungsbögen voneinander unterscheiden.

Die vorstehenden Modelle sind als Implantate dauerhaft in den Bandscheibenräumen verankert. Es kann jedoch insbesondere bei zu kleinflächiger Lastübertragung mittel- bis langfristig zu einer Migration (Verschiebung) der Abschlussplatten in die Wirbelkörper hinein und somit zur Dislokation des gesamten Implantats kommen, wodurch artifizielle Belastungen der Wirbelkörper und der umgebenden Nerven und letztendlich des gesamten Bewegungssegments auftreten können, verbunden mit erneuten Beschwerden des Patienten. Zu diskutieren sind auch die Langzeitbeständigkeit des Polyethylens und bei nicht optimaler Belastung des Polyethylens im Zwischenwirbeiraum die eingeschränkte Beweglichkeit der Bandscheibenprothese. Ungenügend adaptierte Bewegungsumfänge und ungünstige biomechanische Belastungen im Bewegungssegment können unter Umständen zur Beschwerdepersistenz oder später erneut zu Beschwerden des Patienten führen.

Die US 6,706,068 B2 beschreibt hingegen eine Bandscheibenprothese, bestehend aus einem oberen und unteren Teil, wobei die Teile korrespondierend zueinander ausgebildet sind, und kein Zwischenteil als mittlerer Gleitpartner vorhanden ist. An den ineinander greifenden, miteinander artikulierenden Partnern sind unterschiedliche Formgebungen realisiert, so dass es sich um eine zweiteilige Bandscheibenprothese handelt. Diese Formgebung ist allerdings beschränkt auf Strukturen, die entweder Kanten und Ecken aufweisen, so dass auf diese Weise die beiden Prothesenteile miteinander artikulieren; in diesem Fall kann man jedoch nicht mehr von Gleitpartnern sprechen. Des weiteren sind zwei Gleitpartner gezeigt, bei denen der eine Teil konvex ausgebildet ist zur Innenseite der Prothese hin und der andere Gleitpartner entsprechend konkav ausgestaltet ist. Bei dieser Art der Prothese werden jedoch nur eingeschränkt Bewegungen der künstlichen Bandscheibe ermöglicht. Die konkave Ausstülpung entspricht dem Teil einer Kugel mit entsprechendem Krümmungsradius. Die US 6,706,068 B2 zeigt ferner eine zweiteilige Bandscheibenprothese, die auf jedem Gleitpartner konkave und konvexe Teilflächen aufweist, die mit einer entsprechenden konkaven und konvexen Teilfläche des anderen Gleitpartners korrespondieren. Hier entstehen entsprechend der Offenbarung der US 6,706,068 B2 mehrere, fixe Rotationspunkte.

Die US 5,258,031 offenbart eine zweiteilige Bandscheibenendoprothese, wobei die beiden Abschlussplatten über eine kugelige Gelenkverbindung miteinander artikulieren. Das Gelenk ist in Frontalansicht zentral in der Prothese angeordnet. In einer seitlichen Ansicht befindet sich der flächig kleine Artikulationsbereich außerhalb der Mitte. Die Artikulationsflächen sind im Sagittalschnitt sphärisch und im Frontalschnitt plan gestreckt, an den Enden kleinflächig teilsphärisch und anschließend plan schräg, hier jedoch kontaktfrei bei Kontakt der anderen Gelenkbereiche. Beim Seitneigen mit einer Prothese der US 5,258,031 findet daher ein Neigen über die teilsphärische Kante der Artikulationsflächen statt. Ob ein Kontakt der seitlichen Innenflächen der Abschlussplatten entsteht, ist der US 5,258,031 nicht eindeutig zu entnehmen. Die nach außen geöffneten Flächen im beidseits seitlichen Artikulationsbereich, kommen wenigstens bei einer seitlichen Bewegung nicht miteinander in Kontakt. Somit lastet bei einer seitlichen Neigung von Abschlussplatten gemäß der US 5,258,031 der auf den Platten lastende Druck zeitweilig nur auf den teilsphärischen Kanten der Artikulationsflächen. Aufgrund der punkt- bzw. kleinflächigen Druckverteilung beim Seitneigen, sind die Randbereiche der Konvexität/Konkavität einem höheren Verschleiß ausgesetzt. Die Ränder der Prothese nehmen bei den verschiedenen Bewegungen ebenfalls keinen großflächigen Kontakt miteinander auf. Sofern mit einer Prothese nach der US 5,258,031 eine Rotation in einer vertikalen Achse ermöglicht wird, ist nur noch eine beidseits laterale punktuelle Kontaktregion zwischen oberer und unterer Abschlussplatte vorhanden.

Zum technischen Hintergrund von Bandscheibenendoprothesen sei noch auf die EP 1 188 423 A1 verwiesen, die eine arthroplastische Vorrichtung für Zwischenwirbelscheiben beschreibt, umfassend ein Kugelgelenk zwischen einem ersten und einem zweiten Element zum Eingriff in einen ersten und einen zweiten Wirbel in der Wirbelsäule. Ferner sei auf die US 2003/0208273 A1 verwiesen, die eine zweiteilige Bandscheibenendoprothese mit einer konvex-konkaven Artikulationsfläche offenbart.

Der nächstliegende Stand der Technik wird in WO 2004/064692 für eine zweiteilige Prothese und im oben erwähnten EP 0 560 141 B1 für eine dreiteilige Prothese beschrieben.

Die Gegenstände der Ansprüche 1 bzw. 2 unterscheiden sich von diesem Stand der Technik dadurch, dass
der Krümmungsradius der Konvexität (16)
i) aus der Rotation des kleineren Kreisabschnittes entsteht, der sich zwischen den beiden Schnittpunkten einer Sekante (18) mit einem Kreisumfang (19) befindet, wobei die Sekante nicht durch den Mittelpunkt des Kreises geht, und die Rotation um den innerhalb des Kreisumfanges liegenden Abschnitt der Sekante als Rotationsachse stattfindet, und
ii) in sagittaler Ansicht einem Kreisabschnitt entspricht, dessen Radius dem Abstand der Sekante zu dem Kreisumfang aus Punkt i) entspricht.

Desweiteren sind die Merkmale c)b. bis c)d. sowie d) nicht den Dokumenten D1 bzw. D2 zu entnehmen.

Ausgehend von diesem Stand der Technik ist es Aufgabe der vorliegenden Erfindung, eine Bandscheibenendoprothese für den totalen Bandscheibenersatz zur Verfügung zu stellen, bei der das Ausmaß der Bewegung an die Anatomie und Biomechanik der Lenden- und der Halswirbelsäule gezielt angepasst werden kann, wobei der auf den Gleitpartnern lastende Druck bei endgradigem Neigen möglichst großflächig verteilt wird.

Gelöst wird diese Aufgabe durch die Merkmale der selbstständigen Ansprüche 1 und 2. Die Erfindung sieht zwei unterschiedliche Arten einer Bandscheibenendoprothese vor, nämlich eine funktionell zweiteilige und eine funktionell dreiteilige Prothese.

Die funktionell zweiteilige Prothese nach Anspruch 1 zeichnet sich dadurch aus, dass
a) ein erster Gleitpartner derart ausgebildet ist, dass die zur Verbindung mit einem Wirbelkörper entgegengesetzte Seite eine konvexe Krümmung (Konvexität) aufweist, und
   a. der Krümmungsradius der Konvexität
      i. in frontaler und transversaler Ansicht identisch ist und aus der Rotation des kleineren Kreisabschnittes entsteht, der sich zwischen den beiden Schnittpunkten einer Sekante mit einem Kreisumfang befindet, wobei die Sekante nicht durch den Mittelpunkt des Kreises geht, und die Rotation um den innerhalb des Kreisumfanges liegenden Abschnitt der Sekante als Rotationsachse stattfindet, und
      ii. in sagittaler Ansicht einem Kreisabschnitt entspricht, dessen Radius dem Abstand der Sekante zu dem Kreisumfang aus Unterpunkt a) a. i. entspricht, und
   b. die Konvexität von einem Rand umschlossen wird, und
b) ein zweiter Gleitpartner auf der Innenseite mit einer konkaven Artikulationsfläche (Konkavität) ausgebildet ist, und die Geometrie der Konkavität dadurch definiert ist, dass
   a. diese eine zur Konvexität des ersten Gleitpartners korrespondierende Ausnehmung aufweist, welche
   b. von einem Rand umschlossen wird, und
c) die Ränder beider Gleitpartner
   a. einen sich nach außen hin öffnenden Winkel (Öffnungswinkel) in Bezug zueinander aufweisen, wobei
   b. sich die Öffnungswinkel wenigstens im mittigen Frontalschnitt zum mittigen Sagittalschnitt durch die unterschiedliche Neigung der Ränder unterscheiden, um den maximal möglichen Flächenkontakt der Ränder beim endgradigen Bewegen der Gleitpartner zu ermöglichen, und
   c. die unterschiedlichen Neigungen der Ränder fließend ineinander übergehen,
   d. wobei bei gleichen Öffnungswinkeln in einer vertikalen Schnittebene beidseits der Artikulationsfläche die Neigungen der Ränder gleich oder unterschiedlich sind, und
d) in dorsoventraler Richtung der Bewegungswinkel größer ist als in laterolateraler Richtung, welcher aus den unterschiedlichen Krümmungsradien sagittal zu frontal resultiert, und
e) die maximal mögliche Bewegung der Gleitpartner zueinander durch
   a. Krümmungsradius und Höhe der Konvexität in Bezug zum jeweiligen Rand, und
   b. die Ausgestaltung der jeweils korrespondierenden Konkavität, insbesondere Höhe in Bezug zum jeweiligen Rand und Form in Bezug zur korrespondierenden Konvexität, und
   c. die schräg oder waagerecht verlaufenden umgebenden Ränder der Konvexität und Konkavität
festgelegt wird.

Die funktionell dreiteilige Prothese nach Anspruch 2 zeichnet sich dadurch aus, dass
a) der mittlere Gleitpartner auf Ober- und Unterseite eine konvexe Krümmung (Konvexität) aufweist, und der Krümmungsradius der Konvexität auf Ober- und Unterseite
   a. in frontaler und transversaler Ansicht identisch ist und aus der Rotation des kleineren Kreisabschnittes entsteht, der sich zwischen den beiden Schnittpunkten einer Sekante mit einem Kreisumfang befindet, wobei die Sekante nicht durch den Mittelpunkt des Kreises geht, und die Rotation um den innerhalb des Kreisumfanges liegenden Abschnitt der Sekante als Rotationsachse stattfindet, und
   b. in sagittaler Ansicht einem Kreisabschnitt entspricht, dessen Radius dem Abstand der Sekante zu dem Kreisumfang aus a) a. entspricht, und
b) oberer und unterer Gleitpartner mit einer konkaven inneren Artikulationsfläche (Konkavität) ausgebildet sind und die Geometrie der Konkavität des oberen und unteren Gleitpartners jeweils dadurch definiert ist, dass diese jeweils eine zur Konvexität der Ober- oder Unterseite des mittleren Gleitpartners korrespondierende Ausnehmung aufweist, welche jeweils von einem Rand umschlossen wird, und
c) die Ränder der Gleitpartner einen sich nach außen hin öffnenden Winkel (Öffnungswinkel) in Bezug zueinander aufweisen, wobei
   a. sich die Öffnungswinkel wenigstens im mittigen Frontalschnitt zum mittigen Sagittalschnitt durch die unterschiedliche Neigung der Ränder unterscheiden, um den maximal möglichen Flächenkontakt der Ränder beim endgradigen Bewegen der Gleitpartner zu ermöglichen, und
   b. die unterschiedlichen Neigungen der Ränder fließend ineinander übergehen,
   c. wobei bei gleichen Öffnungswinkeln in einer vertikalen Schnittebene beidseits der Artikulationsfläche die Neigungen der Ränder gleich oder unterschiedlich sind, und
d) in dorsoventraler Richtung der Bewegungswinkel größer ist als in laterolateraler Richtung, welcher aus den unterschiedlichen Krümmungsradien sagittal zu frontal resultiert, und
e) die maximal mögliche Bewegung der Gleitpartner zueinander durch
   a. Krümmungsradius und Höhe der Konvexitäten, die Ausgestaltung der jeweils korrespondierenden Konkavität, insbesondere Höhe in Bezug zum jeweiligen Rand und Form in Bezug zur korrespondierenden Konvexität, und
   b. die schräg oder waagerecht verlaufenden umgebenden Ränder der Konkavität
festgelegt wird.

Beiden Prothesen ist gemeinsam, dass sie aus artikulierenden Gleitpartnern bestehen, von denen der jeweils obere Gleitpartner fest mit einem oberen Wirbelkörper und der jeweils untere Gleitpartner fest mit einem unteren Wirbelkörper verbunden ist, und wobei die Gleitpartner auf ihren zueinander gerichteten Innenseiten mit ineinandergreifenden Artikulationsflächen ausgebildet sind. Oberer und unterer Gleitpartner einer dreiteiligen Prothese sowie die beiden Gleitpartner einer zweiteiligen Prothese fungieren gleichzeitig als Abschlussplatten, welche Mittel aufweisen, die zur Verbindung mit einem oberen bzw. unteren Wirbelkörpern dienen.

Wegen der engen anatomischen Raumverhältnisse ist die zweiteilige Bandscheibenendoprothese in erster Linie für die Halswirbelsäule vorgesehen. Die zweiteilige Prothese kann aber auch für die Lendenwirbelsäule vorteilhaft sein wegen der modellimmanenten Stabilität bei Prothesenimplantationen in mehrere übereinander befindliche Bandscheiben. Die dreiteilige Bandscheibenendoprothese hat den Vorteil, dass das Transversalgleiten zweier benachbarter Wirbel nur minimal ist, mit dadurch hervorgerufener vorteilhafter Adaptation an die Biomechanik des Bewegungssegments insbesondere der Lendenwirbelsäule. Mit der dreiteiligen Prothese kann zudem das inkonstante Rotationszentrum simuliert werden.

Im Zusammenhang mit der vorliegenden Erfindung werden die drei Körperachsen durch die folgenden Begriffe bezeichnet: Ein Sagittalschnitt oder eine Ansicht in der sagittalen Ebene erlaubt eine Seitenansicht, da die zugrunde liegende Schnittebene senkrecht von vom nach hinten verläuft. Für die Angabe "vom" wird auch "ventral" und für die Angabe "hinten" analog "dorsal" verwendet, da dies die Orientierung einer Prothese im Körper anzeigt. Ein "Frontalschnitt" oder die "frontale Ebene" ist ein senkrechter Querschnitt von einer Seite zur anderen Seite. Für die Angabe "seitlich" wird auch der Begriff "lateral" verwendet. Sowohl Sagittal- als auch Frontalschnitt sind Vertikalschnitte, da sie entlang einer vertikalen Ebene verlaufen, jedoch um 90 Grad versetzt zueinander orientiert sind. Eine Ansicht in "transversaler Ebene" oder ein "Transversalschnitt" erlaubt eine Aufsicht auf die Prothese, da es sich um einen Horizontalschnitt handelt.

In Verbindung mit der Beschreibung und Darstellung der vorliegenden Erfindung wird unter einer Artikulationsfläche der Bereich von Gleitpartnern verstanden, der aus den gekrümmten konvexen und konkaven Teilen der Oberflächen besteht, welche in Kontakt kommen und miteinander oder aufeinander gleiten bzw. artikulieren. Aus diesem Grund wird für Artikulationsfläche auch gleichbedeutend die Bezeichnung Gleitfläche verwendet.

Der Begriff korrespondierend bezeichnet im Zusammenhang mit artikulierenden Gleitflächen nicht ausschließlich kongruente konvexe und konkave Flächen, die miteinander artikulieren. Vielmehr werden damit auch miteinander artikulierende Gleitflächen bezeichnet, deren Oberflächen nicht vollständig kongruent sind. Derartige "Abweichungen" bzw. Toleranzen bezüglich der Gleitflächen artikulierender Gleitpartner können zum Einen durch die gewählten Materialien und Formen bedingt sein. Andererseits kann es aber auch beabsichtigt sein, dass Konvexität und damit artikulierende Konkavität nicht vollständig kongruent sind, um beispielsweise die zueinander gewünschten Bewegungsmöglichkeiten der Artikulationspartner gezielt vorgeben zu können.

Beiden erfindungsgemäßen Prothesen ist gemeinsam, dass die laterolateral und dorsoventral möglichen Bewegungsausschläge verschieden groß sind und die resultierenden Winkel inkl. der Rotation um eine gedachte Vertikalachse in ihrer Größe definiert werden können.

Die unterschiedlichen seitlichen und dorsoventralen Bewegungswinkel einer erfindungsgemäßen Bandscheibenendoprothese ergeben sich aus der erfindungsgemäßen Gestaltung der konkav-konvexen Artikulationsflächen und stehen zueinander in Beziehung, da vorteilhafterweise die zugrunde liegenden Krümmungsradien durch einen einzigen geometrischen Zusammenhang gemäß den Unterpunkten a) a. aus Anspruch 1 und a) aus Anspruch 2 definiert sind. Die daraus resultierenden konvexen Oberflächen haben im Frontalschnitt immer einen größeren Radius als im Sagittalschnitt. Ein vollständiger Rotationskörper, gemäß den Merkmalen der selbstständigen Ansprüche 1 a) a. und 2 a) hat, jeweils ohne einen Rand, die Form eines "A-meriqan Football" oder einer Spindel, bei der sich jeweils von beiden Seiten zur Mitte hin der Durchmesser gleichartig kontinuierlich vergrößert.

Grundsätzlich sind die Krümmungsradien der Konvexität einer erfindungsgemäßen Bandscheibenendoprothese im Sagittalschnitt immer kleiner als jeder Krümmungsradius in einem Frontal- oder Transversalschnitt. Aus diesem Zusammenhang resultieren für die seitliche Bewegung geringere Bewegungswinkel als für die Extension/Flexion, was auch bei den Bewegungswinkein einer natürlichen Bandscheibe der Fall ist. Somit nähern sich die durch eine erfindungsgemäße Bandscheibenendoprothese ermöglichten Bewegungswinkel dem Bewegungsausmaß der natürlichen Bandscheibe an.

Ein weiterer Vorteil einer erfindungsgemäßen Bandscheibenendoprothese ist, dass diese, zusätzlich zu den an die natürlichen Bewegungsausmaße angenäherten Bewegungswinkeln, die Rotation weich durch eine flächige Kontaktzone begrenzt. Im Gegensatz zu den bisher bekannten Bandscheibenendoprothesen mit entweder einem kleinen oder nahezu punktförmigem festen Anschlag zur Begrenzung der Rotation, oder zu den Bandscheibenprothesen, deren konvexe Oberflächen aus einer Kugelkappe entstammen mit Übergang in eine beispielsweise ellipsoide Form, bei der es ebenfalls zur sehr kleinflächigen bzw. punktförmigen Rotationsbremsung kommt. Durch die erfindungsgemäße Gestaltung der Konvexitäten und der korrespondierenden Konkavitäten ist eine Schonung der artikulierenden Oberflächen gewährleistet, da die Gleitpartner nicht in einem solchen Ausmaß gegeneinander "verdreht" werden können, dass diese nur noch über einzelne Punkte miteinander in Kontakt sind, welche das gesamte auf den oberen und unteren Gleitpartnern lastende Gewicht tragen müssen. Dadurch wird das Material bzw. die Beschichtung der Gleitpartner weniger belastet, wodurch eine erfindungsgemäße Bandscheibenendoprothese auch deutlich langlebiger ist als die aus dem Stand der Technik bekannten Prothesen.

Neben den Vorteilen, die sich aus der erfindungsgemäßen Formgebung der konvex-konkaven Teile der Artikulationsflächen ergeben, weisen die erfindungsgemäßen Bandscheibenendoprothesen aber noch weitere Vorteile auf. Bei einer zwei- und dreiteiligen erfindungsgemäßen Bandscheibenendoprothese sind die Konkavitäten von oberem und unterem Gleitpartner jeweils von einem Rand umschlossen, wohingegen sich die Konvexitäten des mittleren Gleitpartners einer dreiteiligen Prothese jeweils über die gesamte Ober- und Unterseite erstrecken, d.h. die Konvexitäten randfrei sind, oder die Konvexitäten jeweils von einem Rand umgeben sind, dessen Breite gleich oder unterschiedlich ist.

Unter einem Rand soll im Sinne der vorliegenden Erfindung eine Fläche verstanden werden, welche sich zwischen Außenkante des jeweiligen Gleitpartners und Konvexität(en) bzw. Konkavität(en) befindet. Die Ränder der jeweiligen Gleitpartner verlaufen waagerecht und/oder schräg und weisen vorzugsweise eine plane Oberfläche auf. Wesentlich für die Gestaltung der Oberfläche der Ränder ist es, dass es bei einer endgradigen Neigung der Gleitpartner zueinander, zu einem möglichst großflächigen Lückenschluss zwischen den Rändern der Gleitpartner kommt. Sofern die Ränder keine plane Oberfläche aufweisen, sind diese jedenfalls so gestaltet, das es bei einem Lückenschluss zu einem möglichst großflächigen Kontakt der Ränder kommt.

In einer bevorzugten Ausführungsform ist die Höhe der Ränder im unmittelbaren Übergangsbereich der artikulierenden Fläche zur Fläche des Randes rund um die Konvexität bzw. Konkavität unterschiedlich gestaltet. Die Unterschiede in der Höhe des Randes können einerseits einer Anpassung der jeweils maximal möglichen Bewegung der Gleitpartner zueinander dienen. Andererseits können - zum Teil minimale - Unterschiede in der jeweiligen Höhe des Randes, beispielsweise dorsoventral zu laterolateral, auch fertigungstechnisch bedingt sein. Erfindungsgemäß kann die Höhe der Ränder um Konvexität(en) und Konkavität(en), insbesondere auch im direkten Übergangsbereich der artikulierenden Fläche zur Fläche des Randes, aber auch gleich sein und es gibt keine Unterschiede zwischen der Ausgestaltung der jeweiligen Höhe in dorsoventraler zur lateralen Richtung.

Die Ränder der Konvexität(en) und Konkavität(en) weisen ohne Neigung der Gleitpartner zueinander in jeder vertikalen Schnittebene immer einen nach außen offenen Winkel (Öffnungswinkel) auf. Die maximalen Neigungswinkel werden durch den Kontakt des Übergangsbereiches zwischen Konkavität(en) und den die Konkavität(en) umgebenden Rand mit dem Übergangsbereich zwischen korrespondierender/n Konvexität(en) und bei Vorhandensein eines Randes dem die Konvexität(en) umgebenden Rand begrenzt. Dieser Kontakt ist zwar limitierend für die weitere Bewegung der Gleitpartner zueinander, es ist aber nicht der einzige Bereich außerhalb der konkav-konvexen Artikulationsfläche(n), welcher bei endgradiger Neigung in Kontakt kommt. So sind die Ränder der Gleitpartner bis zu deren äußerer Begrenzung derart ausgestaltet, dass diese ebenfalls an dem Lückenschluss beteiligt sind. Dazu haben die Ränder ventral und dorsal aufgrund der größeren Neigungsmöglichkeit nach ventral und dorsal und der somit sonst verbleibenden Lücke ventral und dorsal bei endgradiger Neigung einen größeren Öffnungswinkel als lateral mit fließendem Übergang bei unterschiedlich hohen Randbereichen, so dass bei endgradiger Neigung ein Lückenschluss der Ränder möglich ist, wobei der Lückenschluss je nach Bewegungsrichtung und Richtung der Ansicht vollständig oder unvollständig ist.

Durch diese erfindungsgemäße Maßnahme wird die Fläche bei einem Lückenschluss vergrößert, auf weiche sich der Druck verteilen kann, der bei einer Neigung der Prothese bis zum Anschlag auftritt. Da dieser Druck durch einen flächigen Kontakt aufgenommen wird und nicht nur durch punktförmige Kontaktflächen, werden die miteinander in Kontakt befindlichen Oberflächen zusätzlich vor Abnutzung geschützt, wodurch die Prothese deutlich langlebiger wird.

Bezüglich des Materials ist bei einer erfindungsgemäßen Bandscheibenendoprothese vorgesehen, dass die Gleitpartner einstückig ausgebildet sind oder wenigstens ein Gleitpartner aus wenigstens zwei fest oder fest, aber reversibel miteinander verbundenen Teilen besteht, wobei entweder die Konvexität(en) und/oder Konkavität(en) den Teil ausmachen, der fest oder fest, aber reversibel mit dem jeweiligen Gleitpartner verbunden ist, oder Konvexität(en) und/oder Konkavität(en) an der Basis geeignete Mittel für eine feste oder feste, aber reversible Verbindung aufweisen, wobei miteinander verbundene Teile aus gleichen oder verschiedenen Materialien bestehen oder die Oberflächen der Teile gleich oder verschieden beschichtet sind. Als geeignete Mittel für eine Verbindung sind erfindungsgemäß Anpassungen der Form der miteinander zu verbindenden Teile vorgesehen, wie beispielsweise flächige Verbreiterungen, die Teil des Randes oder der gesamte Rand sind, oder Ausnehmungen. Als Teile, die abhängig von der jeweiligen Ausführungsform miteinander verbunden sein können, sind der jeweilige Gleitpartner und/oder Konvexität und/oder Konkavität sowie der Rand vorgesehen. Bei einem mittleren Gleitpartner ist zudem vorgesehen, dass dieser erst aus der Verbindung der jeweiligen Teile entsteht.

Sofern eine erfindungsgemäße Bandscheibenendoprothese aus fest oder fest, aber reversibel miteinander verbundenen Teilen besteht, ist für die Verbindung zwischen Gleitpartner und Konvexität(en) oder Konkavität(en) vorzugsweise eine Nut/Feder-Verbindung, eine Führungsschiene und korrespondierende Ausnehmung, ein Schnappmechanismus, Kleben oder Verschrauben vorgesehen.

Bei einer dreiteiligen erfindungsgemäßen Bandscheibenendoprothese ist erfindungsgemäß auch vorgesehen, dass oberer und unterer Gleitpartner aus dem gleichen Material bestehen oder gleich beschichtet sind und der mittlere Gleitpartner aus einem anderen Material gefertigt oder anders beschichtet ist.

Die Gleitpartner werden aus in der Implantattechnik bewährten Materialien gefertigt; beispielsweise bestehen der obere und untere Gleitpartner aus nichtrostendem Metall und der mittlere Gleitpartner aus medizinischem Polyethylen. Andere Materialkombinationen sind denkbar. Die Verwendung anderer alloplastischer Materialien, die auch bioaktiv sein können, ist ebenfalls vorgesehen. Die Gleitpartner sind an den zueinander gerichteten Berührungsflächen hochglanzpoliert, um den Abrieb zu minimieren (iow-friction-Prinzip). Im übrigen ist auch eine Beschichtung der einzelnen Gleitpartner mit geeigneten Materialen vorgesehen. Bevorzugt sind folgende Materialien vorgesehen: Titan, Titanlegierungen oder Titancarbid, Legierungen aus Kobalt und Chrom oder anderen geeigneten Metallen, Tantal oder geeignete Tantalverbindungen, geeignete keramische Materialien sowie geeignete Kunststoffe oder Verbundwerkstoffe.

In einer bevorzugten erfindungsgemäßen Ausführungsform einer dreiteiligen Prothese ist vorgesehen, dass die Krümmungsradien der Konvexitäten auf Ober- und Unterseite des mittleren Gleitpartners sowie der korrespondierenden Konkavitäten in oberem und unterem Gleitpartner identisch sind. Bei identisch gekrümmten Konvexitäten auf Ober- und Unterseite ist abhängig von der Ausführungsform ferner vorgesehen, dass die maximale Höhe der Konvexitäten des mittleren Gleitpartners auf Ober- und Unterseite im gleichen oder unterschiedlichen Ausmaß geringer als bei einer gemeinsamen Rotationsachse eines Kreisabschnittes nach Unterpunkt 2 a) a. ist und bei vorhandenem Rand die Höhe des Randes um einen gleichen Betrag wie die Höhe der Konvexität(en) verringert ist oder die Höhe des Randes gleich bleibt oder different zur Höhenänderung der Konvexität(en) ist, wobei die maximale Höhe der Konvexitäten auf Ober- und Unterseite dadurch gleich oder unterschiedlich ist.

Durch diese erfindungsgemäße Maßnahme wird die Gesamthöhe der Prothese reduziert, da der mittlere Gleitpartner abgeflacht ist. Gleichzeitig werden dadurch die artikulierenden Flächen für eine materialschonende Druckübertragung im Zwischenwirbeiraum vergrößert. Dadurch werden auch Maße bei einer derart ausgestalteten Prothese erreicht, die es ermöglichen, diese in physiologisch besonders schmale Bandscheibenräume einzusetzen. Zudem bietet eine derartige Ausführungsform die Möglichkeit, die Höhe des mittleren Gleitpartners zu variieren und so eine in der Höhe angepasste Prothese zu erhalten.

Ferner sind Ausführungsformen vorgesehen, bei denen sich die Krümmungsradien der Konvexitäten auf Ober- und Unterseite des mittleren Gleitpartners und die korrespondierenden Konkavitäten in oberem und unterem Gleitpartner unterscheiden. Dadurch werden die Möglichkeiten der Adaptation der Bewegungsmaße einer erfindungsgemäßen Bandscheibenendoprothese an die physiologischen Bewegungsmaße erweitert. Auch bei unterschiedlichen Krümmungsradien der Konvexitäten auf Ober- und Unterseite eines mittleren Gleitpartners ist eine Ausführungsform vorgesehen, bei der die maximale Höhe der Konvexitäten des mittleren Gleitpartners auf Ober- und Unterseite im gleichen oder unterschiedlichen Ausmaß geringer ist, als bei Rotationsachsen nach Unterpunkt 2 a) a. von zwei unterschiedlich gekrümmten Kreisabschnitten und/oder bei vorhandenem Rand die Höhe des Randes um einen gleichen Betrag wie die Höhe der Konvexität(en) verringert ist oder die Höhe des Randes gleich bleibt oder different zur Höhenänderung der Konvexität(en) ist, wobei die maximale Höhe der Konvexitäten auf Ober- und Unterseite dadurch gleich oder unterschiedlich ist.

Bei dieser "kompakten" Ausführungsform einer erfindungsgemäßen dreiteiligen Bandscheibenendoprothese wird ein Herausgleiten des mittleren Gleitpartners einerseits durch die bewegungsadaptierten Höhen der Konvexität auf Ober- und Unterseite und der korrespondierenden Konkavitäten ab dem Rand rund um die Artikulationsflächen und andererseits durch den Lückenschluss der Ränder der Gleitpartner bei endgradiger Neigung verhindert. Die Konvexitäten sind derart gestaltet, dass sie tief genug in die artikulierenden Konkavitäten "eingreifen". Ein ausreichendes Aufspreizen der gesamten Prothese postoperativ, welches für das Herausgleiten des mittleren Gleitpartners Voraussetzung wäre, ist somit nicht möglich.

Bei einer erfindungsgemäßen zwei- oder dreiteiligen Bandscheibenendoprothese ist jeweils ein maximaler Öffnungswinkel bei einseitigem Lückenschluss der Gleitpartner bei Extension oder Flexion zwischen 6 und 10 Grad und bei einseitigem lateralen Lückenschluss zwischen 3 und 6 Grad vorgesehen. Adaptiert an die Lenden- bzw. Halswirbelsäule können die konkreten maximalen Bewegungsmaße konstruktiv angepasst werden, ohne für jede einzelne Bandscheibe eine "eigene Prothese" vorzusehen. Die Öffnungswinkel entsprechen der natürlichen Segmentbeweglichkeit und werden durch eine geeignete Wahl der Konvexität(en) und Konkavität(en) in Zusammenhang mit der Ausgestaltung der diese umgebenden Ränder erreicht (s.o.). Zum Ausgleich von Toleranzen im Bewegungssegment werden zusätzliche 3 Grad in jede Bewegungsrichtung einbezogen.

Sowohl bei einer funktionell zwei- als auch bei einer funktionell dreiteiligen Bandscheibenendoprothese wird bei Kongruenz von Konvexität(en) und Konkavität(en) artikulierender Gleitpartner eine Rotation um eine gedachte zentrale Vertikalachse abgebremst.

In einer weiteren Ausführungsform einer zwei- oder dreiteiligen Bandscheibenendoprothese ist vorgesehen, dass die Konkavität jeweils lateral breiter als die zugehörige Konvexität ausgestaltet ist. Die konkave Ausnehmung ist lateral verbreitert, wobei die Verbreiterung abgerundet ist und sich die Form der Abrundung an der Form der Konvexität orientiert. Es kann sich auch um den konkaven Ausschnitt eines Torus handeln, der laterolateral den gleichen Krümmungsradius aufweist wie die Konvexität laterolateral. Durch diese erfindungsgemäße Ausführungsform wird eine begrenzte Rotationsbewegung der Gleitpartner zueinander ermöglicht, die abhängig von dem Ausmaß der lateralen Verbreiterung eine Rotation um eine gedachte zentrale Vertikalachse von bis zu 3 Grad für die Lendenwirbelsäule und bis zu 6 Grad für die Halswirbelsäule nach jeder Seite ermöglicht. Zum Ausgleich von Toleranzen im Bewegungssegment werden zusätzliche 2 Grad nach jeder Seite einbezogen.

Ist eine Konkavität lateral breiter als die mit ihr artikulierende Konvexität ausgestaltet, so kann sich diese in der konkaven Ausnehmung in Richtung einer Diagonalen verdrehen. Je nach Ausgestaltung der lateralen Verbreiterung ist eine begrenzte Drehung der Gleitpartner gegeneinander zu erreichen, bei der sich die Gesamthöhe der Prothese nicht verändert. In jedem Fall wird die Drehung der Konvexität aber durch den Widerstand begrenzt, der aus einem Aufgleiten der Prothese auf die randnahe Artikulationsfläche der Konkavität resultiert.

In einer alternativen Ausführungsform der Konkavitäten einer erfindungsgemäßen Bandscheibenendoprothese ist vorgesehen, dass die zu einer Konvexität korrespondierende Konkavität bis hin zu einer hohlkugelförmigen Ausnehmung ausgebildet ist, wobei der Krümmungsradius dem größeren Krümmungsradius der zugehörigen Konvexität entspricht.

Bei derart ausgestalteten Artikulationspartnern ist theoretisch eine freie Rotation der Gleitpartner, bei einer dreiteiligen Prothese des mittleren Gleitpartners, denkbar. Daher ist diese Ausführungsform insbesondere für zweiteilige Prothesen vorgesehen, da bei diesen eine freie Rotation des mittleren Gleitpartners, aufgrund ihrer Verbindung mit einem oberen bzw. unteren Wirbelkörper, ausgeschlossen ist.

In einer weiteren bevorzugten Ausführungsform einer erfindungsgemäßen zwei- oder dreiteiligen Bandscheibenendoprothese ist ein "Versatz" von Konvexität(en) und korrespondierender(n) Konkavität(en) um bis zu 4 mm vom mittigen Sagittalschnitt nach dorsal vorgesehen.

Ein nach dorsal versetztes Rotationszentrum entspricht vor allem der physiologischen Situation im Übergang zwischen der Lendenwirbelsäule und dem Kreuzbein und andererseits werden so parallel die der physiologischen Situation entsprechenden Unterschiede zwischen den möglichen Neigungswinkeln bei der Extension und Flexion erreicht.

Ferner ist vorgesehen, dass die Ränder der Gleitpartner außen rechtwinklig, anderweitig winklig, gekrümmt oder kombiniert gerade, gekrümmt und/oder winklig abgeschlossen sind. Insbesondere bei einer dreiteiligen Prothese ist auch eine Ausführungsform der Prothese mit Rand des mittleren Gleitpartners denkbar, bei weicher Ober- und Unterseite des mittleren Gleitpartners im äußeren Randbereich einfach rechtwinklig oder gekrümmt miteinander abschneiden und die Randbreite gleich oder anders als bei oberem und unterem Gleitpartner ausgestaltet ist. Somit wird der mittlere Gleitpartner auch bei endgradiger Neigung noch zwischen dem oberen und unteren Gleitpartner verbleiben, da beim Lückenschluss die Konkavität des oberen und/oder unteren Gleitpartners die jeweils korrespondierende Konvexität des mittleren Gleitpartners über deren maximale Höhe hinaus umfasst.

In einer weiteren Ausführungsform einer dreiteiligen Bandscheibenendoprothese ist vorgesehen, dass die Höhe des Randes vom mittleren Gleitpartner ab dem Übergangsbereich zwischen der Konvexität und dem Rand bis zum äußeren Randbereich teilweise oder insgesamt kontinuierlich größer wird, ohne dass sich die Größe der Öffnungswinkel infolge Anpassung der Randhöhe des oberen und unteren Gleitpartners ändert. Diese "Schwalbenschwanzform" vom Rand des mittleren Gleitpartners erhöht dessen Sicherheit entgegen einer Dislokation.

Erfindungsgemäß ist bei dreiteiligen Prothesen auch eine Form von oberem und/oder unterem Gleitpartner vorgesehen, bei dem äußere Randbereiche vollständig oder partiell hakenförmig, rechtwinklig, anderweitig winklig, gekrümmt oder in Kombinationen davon in Richtung des anderen äußeren Gleitpartners ausgestaltet sind. Der Rand des mittleren Gleitpartners ist dort bei dieser Ausführungsform schmaler, so dass der mittlere Gleitpartner partiell oder vollständig von den Vorrichtungen eines oder beider äußerer Gleitpartner eingefasst wird, um ein Herausgleiten des mittleren Gleitkörpers zu verhindern. Vorteilhafterweise ist der Rand des mittleren Gleitpartners derart an die Randform eines äußeren Gleitpartners angepasst, dass bei einem endgradigen Lückenschluss eine möglichst große Fläche der artikulierenden Gleitpartner in Kontakt kommt.

Weiterhin ist es erfindungsgemäß vorgesehen, dass bei einer dreiteiligen Prothese beim mittleren Gleitpartner mit Rand zur zusätzlichen Sicherung gegen ein Herausgleiten, Abgleiten bzw. Herausrutschen (Luxation) bei Lückenschluss aller drei Gleitpartner, ein Anschlag Teil des äußeren Randbereichs des mittleren Gleitpartners ist, der außerhalb des oberen und/oder unteren Gleitpartners angeordnet ist, wobei der Anschlag wenigstens auf Ober- oder Unterseite höher ist als der Rand des mittleren Gleitpartners.

Dieser Anschlag zur zusätzlichen Sicherung gegen ein Herausgleiten, Abgleiten bzw. Herausrutschen (Luxation) kann erfindungsgemäß auch derart ausgestaltet sein, dass der Anschlag Teil des Randes des mittleren Gleitpartners ist, wobei der Anschlag auf Ober- und/oder Unterseite höher als der Rand des mittleren Gleitpartners ist und innerhalb einer Nut im Randbereich des oberen und/oder unteren Gleitpartners mit dem notwendigen Spiel für die maximale Gleitbewegung der Gleitpartner geführt wird.

Unter einem Anschlag soll im Sinne der vorliegenden Erfindung eine nach außen gerichtete Fortführung des Randes eines mittleren Gleitpartners verstanden werden, welche aufgrund der jeweiligen Ausgestaltung geeignet ist, ein Herausgleiten des mittleren Gleitpartners aus den Konkavitäten des oberen und unteren Gleitpartners zu verhindern. Ein Anschlag muss den mittleren Gleitpartner dazu nicht vollständig umschließen, da dies zu Einschränkungen der maximalen Beweglichkeit aller Gleitpartner führen kann, sondern gegebenenfalls in definierten Abständen oder gegenüber von Positionen des Randes angeordnet sein, welche für ein Herausgleiten des mittleren Gleitpartners in Frage kommen. Sofern der Anschlag auf Ober- und Unterseite höher als der Rand des mittleren Gleitpartners ist, kann er beispielsweise wie eine Heftzwecke ausgestaltet sein, die mit der Nadelspitze von außen in den Rand des mittleren Gleitpartners gesteckt wurde, so dass der Kopf der Heftzwecke oben und unten über den Rand des mittleren Gleitpartners übersteht und bei einer endgradigen Neigung zur Position der Heftzwecke das Herausgleiten des mittleren Gleitpartners verhindert, indem er an dem oberen und unteren Gleitpartner "anschlägt".

Ist ein Anschlag zur Sicherung gegen ein Herausgleiten Teil des Randes der Gleitpartner, so ist die Höhe der Konvexität unter Beachtung der Anatomie und Materialeigenschaften lediglich abhängig von den gewünschten maximalen Neigungswinkeln, auf welche diese auch Einfluss hat (s.o.).

Ein Anschlag zur Sicherung des mittleren Gleitpartners bei einer dreiteiligen Prothese ist vorteilhafterweise derart gestaltet, dass er bei einer endgradigen Neigung der Gleitpartner ebenfalls an dem Lückenschluss des Randes beteiligt ist. Dadurch kommt dem Anschlag nicht nur eine Sicherungsfunktion zu, sondern er dient zusätzlich der Vergrößerung der mit Druck belasteten Flächen im Falle der endgradigen Neigung der Gleitpartner, deren Vorteile bereits beschrieben wurden. Die Möglichkeit einer derartigen Gestaltung hängt aber entscheidend von der Außenform des oberen und unteren Gleitpartners und der jeweiligen Randbreite von Konvexität und Konkavität ab.

Ferner ist bei der erfindungsgemäßen Bandscheibenendoprothese vorgesehen, dass sich der Außenumfang des oberen und unteren Gleitpartners in transversaler Ansicht von dorsal nach ventral (Lendenwirbelsäule) oder von ventral nach dorsal (Halswirbelsäule) verjüngen kann. Diese Verjüngung des Außenumfangs des oberen und unteren Gleitpartners kann lateral jeweils als identische Krümmung ausgebildet sein und ist bevorzugt ein Teilausschnitt eines Kreises. Fläche und Form des Außenumfanges des oberen und unteren Gleitpartners können je nach Bedarf gleich oder ungleich sein und so an die jeweilige Größe des Wirbelkörpers, mit dem sie verbunden werden, angepasst werden.

Die sich verjüngende Form der oberen und unteren Gleitpartner entspricht im wesentlichen der für die Prothesenplatten nutzbaren Fläche eines Wirbelkörpers in der Transversalansicht und führt so zu einer optimalen Nutzung der zur Verfügung stehenden Fläche eines Wirbelkörpers zur Verankerung der Gleitpartner mit dem Ziel einer möglichst großflächigen Lastübertragung des auf den Gleitpartnern aufliegenden Druckes.

Ferner sind bei einer erfindungsgemäßen Bandscheibenendoprothese Gleitpartneranpassungen vorgesehen, wobei oberer und/oder unterer Gleitpartner im Frontal- und/oder Sagittalschnitt derart ausgebildet sind, dass die Außen- und Innenseite von oberem und/oder unterem Gleitpartner parallel oder eben nicht parallel zueinander verlaufen. Durch diese erfindungsgemäße Maßnahme kann eine erfindungsgemäße Bandscheibenendoprothese an Wirbelkörperendplatten angepasst werden, welche in Frontalansicht nicht parallel zueinander stehen bzw. in der Sagittalansicht eine optimale Lordose und Gleitflächenstellung zueinander ausbilden sollen.

Weiterhin ist vorgesehen, dass bei einer zwei- und dreiteiligen erfindungsgemäßen Ausführungsform die Konvexität (zweiteilige Prothese) bzw. der mittlere Gleitpartner (dreiteilige Prothese) bezüglich einer gedachten Horizontalen parallel oder nicht parallel ist. Bei einer nicht parallelen Ausführungsform stehen Ober- und Unterseite in einem Winkel in Bezug zur gedachten Horizontale zueinander, wobei bei einem mittleren Gleitpartner der Winkel oben und unten gleich groß oder unterschiedlich sein kann. Die Konvexität(en) und korrespondierende(n) Konkavität(en) sind bei der zwei- und dreiteiligen Prothese in ihrer Oberflächengestaltung symmetrisch oder asymmetrisch. Durch die gewinkelte Konvexität bzw. den gewinkelten mittleren Gleitpartner sind ebenfalls Anpassungen an Asymmetrien des Wirbelkörperzwischenraumes möglich, in welchen die Prothese implantiert wird.

Zur sicheren Implantatverankerung im Zwischenwirbelraum dient eine randständige und/oder flächenhafte Verzahnung der Außenseiten von oberem und unterem Gleitpartner zur Verbindung mit einem oberen bzw. unteren Wirbelkörper. Die Außenseiten selbst sind plan oder konvex geformt und es ist möglich, die Verzahnung oder die wirbelkörperseitige Fläche mit und ohne Verzahnung des oberen und unteren Gleitpartners bioaktiv oder auch stumpf zu beschichten. Um das Risiko einer Fraktur des Wirbelkörpers zu minimieren, ist eine Verankerung mit drei ventral angeordneten und zwei dorsal angeordneten Verankerungszähnen bevorteilt. Alternativ sind durchgehende laterale Zahnreihen bevorteilt, zur besseren Führung des oberen und unteren Gleitpartners beim Einsetzen zwischen den Wirbelkörpern, da die Arbeitszange des Operateurs in die mittlere Lücke zwischen den Zahnreihen greifen kann oder auf Höhe der Zähne in Führungslöcher des oberen und unteren Gleitpartners.

Zur Vereinfachung der Im- oder Explantation der Bandscheibenendoprothese weisen oberer und/oder unterer Gleitpartner in einer weiteren Ausführungsform Vorsehungen für Instrumente auf. Diese Vorsehungen bestehen vorzugsweise aus Löchern oder Ausformungen, in die das jeweils benötigte Instrument des Operateurs eingreifen kann und so ein sicherer Halt des jeweiligen Gleitpartners ermöglicht wird.

Bei einer erfindungsgemäßen Bandscheibenendoprothese sind des weiteren als absolute Maße eine maximale Breite (Frontalansicht) von 14 bis 48 mm, eine maximale Tiefe (Sagittalschnitt) von 11 bis 35 mm und eine maximale Höhe von 4 bis 18 mm vorgesehenen. Diese Maße orientieren sich an den natürlichen Gegebenheiten der Lenden- und Halswirbelsäule und gewährleisten so, dass eine erfindungsgemäße Bandscheibenendoprothese der *in vivo* Situation möglichst nahe kommt.

Ferner sind bei einer erfindungsgemäßen Bandscheibenendoprothese ein oder mehrere röntgenkontrastgebende Markierungen vorgesehen, welche nicht röntgenkontrastgebende Teile der Prothese jeweils unterhalb ihrer Oberfläche enthalten. Dadurch ist es möglich, die Lage dieser Teile einer Bandscheibenendoprothese direkt nach der Implantation auf eine exakte Lage hin zu kontrollieren. Des weiteren ist es möglich, in definierten Zeitabständen durch Röntgen zu überprüfen, ob diese Teile der Prothese sich in ihrer Lage verändert haben bzw. immer noch exakt positioniert sind.

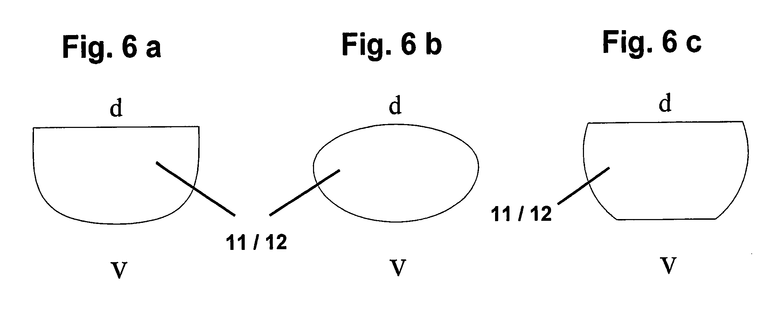

Weitere vorteilhafte Maßnahmen sind in den übrigen Unteransprüchen beschrieben; die Erfindung wird anhand von Ausführungsbeispielen und der nachfolgenden Figuren näher beschrieben; es zeigt:

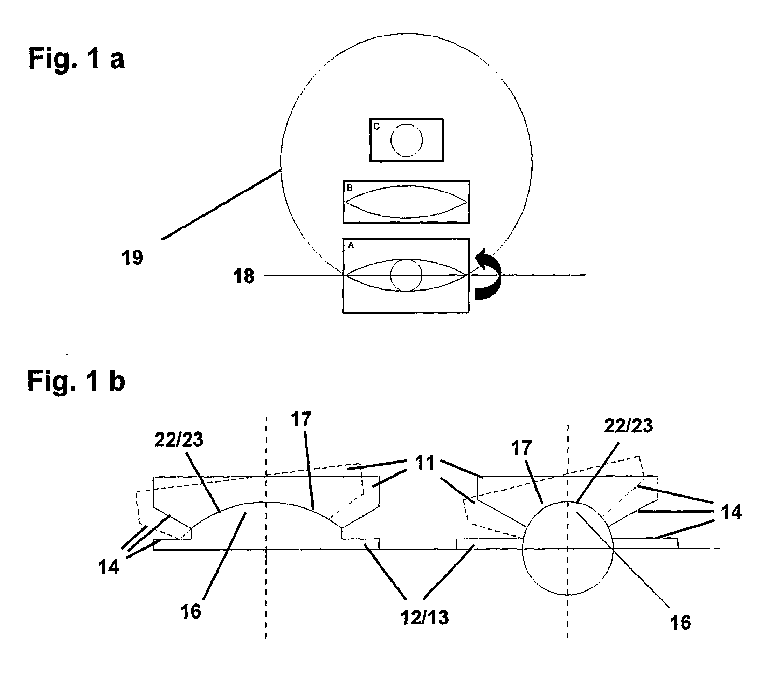

- **Figur** 1 **a**: Schematische Darstellung eines mittleren Gleitpartners mit i- dentisch gekrümmter Ober- und Unterseite, abgeleitet aus ei- nem Kreisumfang und einer Sekante als Rotationsachse:
A: Zusammenhang der maximalen Höhe der Konvexitäten im medianen frontalen und transversalen Schnitt des mittleren Gleitpartners mit dem Durchmesser im medianen Sagit- talschnitt:
B: Konvexität des mittleren Gleitpartners ohne Rand im medi- anen Frontal- und Transversaischnitt
C: Konvexität des mittleren Gleitpartners im medianen Sagit- talschnitt
- **Figur 1 b**: Schematische Ansicht eines Frontalschnittes (links) und Sag ittalschnittes (rechts)
- **Figur 2**: Schematische Frontalansicht einer dreiteiligen erfindungsge- mäßen Bandscheibenendoprothese mit Rand des mittleren Gleitpartners, abgeleitet aus identischen Kreisumfängen:
a: ohne Neigung der Gleitpartner
b: endgradige Neigung der Gleitpartner zur linken Seite
- **Figur 3 a - c**: Schematische Sagittalansicht einer dreiteiligen erfindungsge- mäßen Bandscheibenendoprothese mit Rand des mittleren Gleitpartners
a: ohne Neigung der Gleitpartner
b: endgradige Neigung der Gleitpartner zur linken Seite
c: mit abgeflachtem mittleren Gleitpartner
- **Figur 4 a - d**: Schematische Darstellung einer zweiteiligen erfindungsgemä- ßen Bandscheibenendoprothese. Links ist jeweils die Prothe- se ohne Neigung und rechts mit endgradigem Lückenschluss der Gleitpartner dargestellt:
a: Frontalansicht
b: Sagittalansicht
c: Transversalansicht mit Konkavität
d: Transversalansicht mit lateral verbreiterter Konkavität und mit Konvexität, links ohne, rechts mit Rotation
- **Figur 5 a - g**: Schematische Darstellung einer dreiteiligen erfindungsgemä- ßen Bandscheibenendoprothese mit Rand des mittleren Gleit- partners. Bei der Frontal- und Sagittalansicht ist links jeweils die Prothese ohne Neigung und rechts mit endgradigem Lü- ckenschluss der Gleitpartner dargestellt:
a: Frontalansicht
b: Sagittalansicht
c: Transversalansicht mit Konkavität
d: Transversalansicht mit lateral verbreiterter Konkavität und mit Konvexität, links ohne, rechts mit Rotation
e: Transversalansicht mit nach dorsal versetztem Rotations- zentrum der Konkavität (für die Lendenwirbelsäule)
f: Sagittalansicht mit nach dorsal versetztem Rotationszentrum
g: Transversalansicht mit lateral verbreiterter Konkavität, mit Konvexität und nach dorsal versetztem Rotationszentrum (für die Lendenwirbelsäule), links ohne, rechts mit Rotation der Konvexität in der Konkavität
- **Figur 6 a - c**: Schematische Darstellung verschiedener Formen des oberen und unteren Gleitpartners für die Lendenwirbelsäule

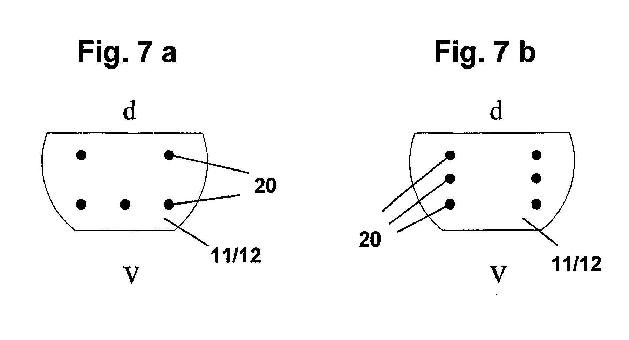

- **Figur 7 a, b**: Schematische Darstellung der Anordnung von Verankerungs- zähnchen auf den Außenseiten des oberen und unteren Gleit- partners für die Lendenwirbelsäule

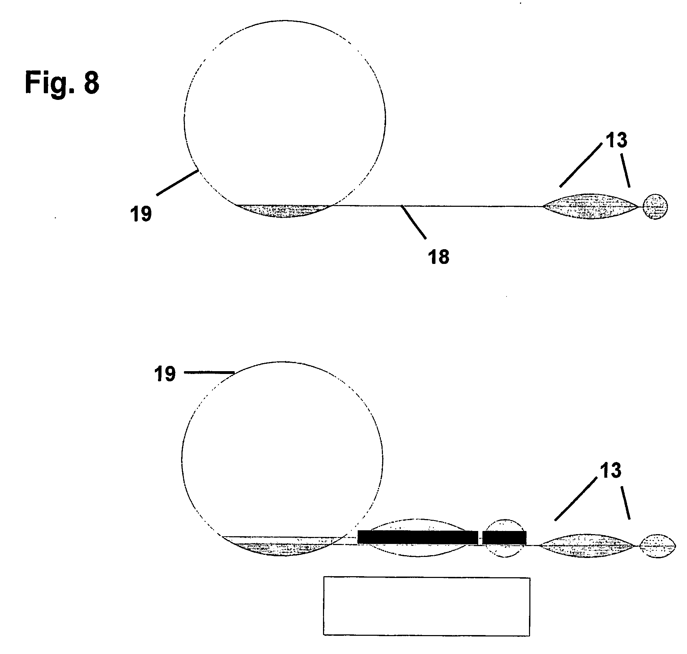

- **Fig. 8**: Schematische Darstellung des mittleren Gleitpartners der er- findungsgemäßen Bandscheibenendoprothese (oben wie Fig. 1a, unten mittlerer Gleitpartner mit frontal und sagittal gleicher Höhe, jedoch sagittal größerer Artikulationsfläche infolge hori- zontal reduziertem symmetrischen Fragment eines ursprüng- lich größeren Gleitpartners)

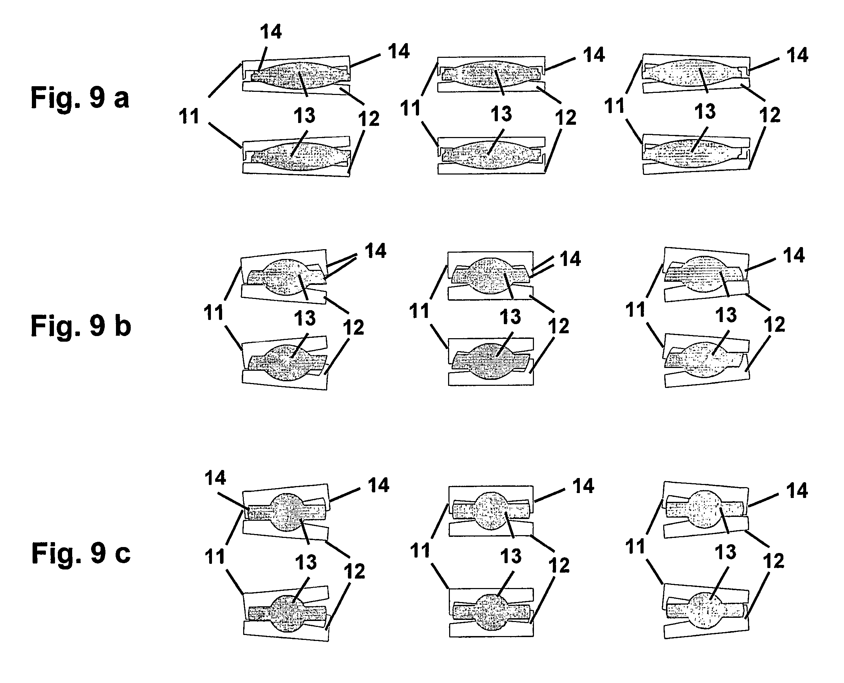

- **Fig. 9 a - c**: Schematische Darstellung von Varianten einer dreiteiligen er- findungsgemäßen Bandscheibenendoprothese mit Rand zur Sicherung des mittleren Gleitpartners durch die Randgestal- tung des oberen und/oder unteren Gleitpartners. Links und rechts ist jeweils die Neigung der Prothese mit endgradigem Lückenschluss der Gleitpartner dargestellt.
a: Frontalansicht
b - c: Sagittalansichten

Figur 1 a, Figurenteil A, zeigt, wie die Krümmung der Oberflächen eines mittleren Gleitpartners mit identischen Ober- und Unterseiten aus einem Kreisumfang 19 abgeleitet wird und aus einer Rotation des kleineren Kreisabschnitts, weiche durch den Pfeil angedeutet ist, um die Sekante 18 entsteht. Teil B der Abbildung zeigt die aus der Rotation resultierende Form, welche in einem Frontal- und Transversalschnitt identisch ist. Im Sagittalschnitt C des Körpers, welcher aus der Rotation des kleineren Kreisabschnittes um die Sekante 18 entsteht, weist dieser jeweils einen kreisförmigen Querschnitt auf. Die Radien im Sagittalschnitt nehmen von der Mitte des geometrischen Körpers nach beidseits lateral kontinuierlich ab.

In Figur 1 b ist links schematisch ein Frontalschnitt einer erfindungsgemäß ausgestalteten Gleitfläche (22, 23) einer zwei- oder dreiteiligen Bandscheibenendoprothese zu sehen. Im rechten Teil von Figur 1 b ist schematisch ein Sagittalschnitt zu sehen. Es ist zu erkennen, dass die maximale Höhe der Konvexität 16 im Frontal- und Sagittalschnitt identisch ist, wobei sich aber die Krümmungsradien in diesen um 90° zueinander versetzten Schnittebenen deutlich voneinander unterscheiden. Mit einer gestrichelten Linie ist ein maximal geneigter oberer Gleitpartner 11 mit Konkavität 17 dargestellt, der mit der Konvexität 16 eines mittleren Gleitpartners 13 einer dreiteiligen oder des unteren Gleitpartners 12 einer zweiteiligen Bandscheibenendoprothese artikuliert. Der Rand 14 welcher die Konvexität 16 umgibt, ist hier zur exakten Darstellung des Winkelzusammenhangs rund um die Konvexität 16 immer gleich hoch ausgestaltet. Wie aus beiden Ansichten der Figur 1 b hervorgeht, ist der Bereich des Übergangs von Konkavität 17 zur Fläche des Randes 14 der Bereich, welcher die maximale Bewegung der Gleitpartner 11, 12, 13 zueinander limitiert, da dieser Bereich zuerst in Kontakt kommt, ohne dass der Rand 14 in dieser schematischen Darstellung beim endgradigen Neigen einen Lückenschluss aufweist. Im Frontalschnitt ergibt sich, abgeleitet aus den unterschiedlichen Krümmungsradien frontal zu sagittal, eine geringere maximale Beweglichkeit der Gleitpartner 11, 12, 13 zueinander, als im Sagittalschnitt. Somit ist bei einer erfindungsgemäß ausgestalteten Konvexität 16 und Konkavität 17 eine stärkere dorsoventrale Neigung der Gleitpartner 11, 12, 13 zueinander möglich als eine Neigung in laterolateraler Richtung.

Damit die Flächen der Ränder 14 in Kontakt kommen, müssen diese zueinander geneigt sein. Dabei müssen die dorsalen und ventralen Ränder 14 für einen Lückenschluss einen größeren Öffnungswinkel haben, als die seitlichen Ränder 14. Die jeweilige Neigung der Ränder 14 dient dazu, um einen Lückenschluss zu erreichen, sie hat allein keinen Einfluss auf die maximale Beweglichkeit der Gleitpartner 11, 12, 13 zueinander. Soweit die Neigung der Ränder des mittleren Gleitpartners die Randhöhe dieses Gleitpartners nach peripher kontinuierlich vergrößert, stabilisiert der Rand den mittleren Gleitpartner entgegen einer Dislokation bei endgradigen Bewegungen der drei Gleitpartner. Die unterschiedlichen Neigungen der Ränder gehen fließend ineinander über. Bei einer erfindungsgemäßen dreiteiligen Bandscheibenendoprothese ist für den mittleren Gleitpartner 13 nicht zwingend ein Rand 14 vorgesehen. Sofern der mittlere Gleitpartner 13 keinen Rand aufweist, sind die Ränder 14 des oberen und unteren Gleitpartners 11, 12 derart ausgestaltet, dass diese bei endgradiger Neigung einen möglichst großflächigen Lückenschluss ermöglichen. Die Konvexitäten 16 eines mittleren Gleitpartners 13 und die Konkavitäten 17 von oberem und unterem Gleitpartner 11, 12 sind, sofern der mittlere Gleitpartner 13 keinen Rand 14 aufweist, so ausgestaltet, das die Konvexitäten 16 tief genug in die Konkavitäten 17 eingreifen, um einerseits ein Herausgleiten des mittleren Gleitpartners 13 zu verhindern und andererseits den Lückenschluss der Ränder 14 zu ermöglichen.

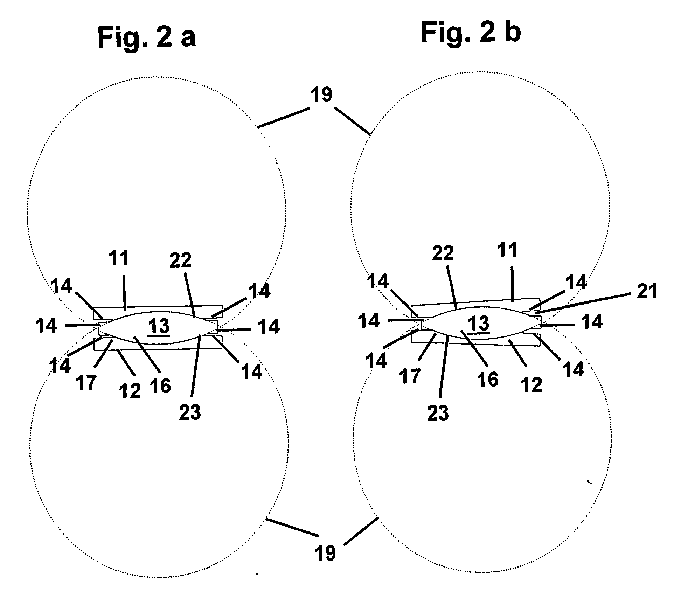

Figur 2 a und b zeigt jeweils eine schematische Frontalansicht einer dreiteiligen erfindungsgemäßen Bandscheibenendoprothese mit Rand des mittleren Gleitpartners abgeleitet aus identischen Kreisumfängen 19. In Figur 2 a ist die Prothese in einer sogenannten Nullstellung dargestellt, in der der obere und untere Gleitpartner (11, 12) sowie mittlere Gleitpartner 13 nicht zueinander geneigt sind. In Figur 2 b ist eine endgradige Neigung der drei Gleitpartner mit Lückenschluss auf der linken Seite dargestellt.

Die Krümmungsradien der Konvexitäten des mittleren Gleitpartners 13 auf Ober- und Unterseite sind identisch. Diese bilden mit den korrespondierenden Konkavitäten des oberen und unteren Gleitpartners 11, 12 jeweils eine obere und eine untere Artikulationsfläche 22, 23. Die Konvexität 16 des mittleren Gleitpartners 13 wird von einem Rand 14 der Konvexität 16 vollständig umschlossen. Die korrespondierende Konkavität wird ebenfalls von einem Rand 14 vollständig umschlossen.

Bei einem einseitig endgradigen Lückenschluss (rechter Teil) vergrößert sich der Öffnungswinkel 21 entsprechend des Lückenschlusses auf der gegenüberliegenden Seite der Konvexität/Konkavität 16,17.

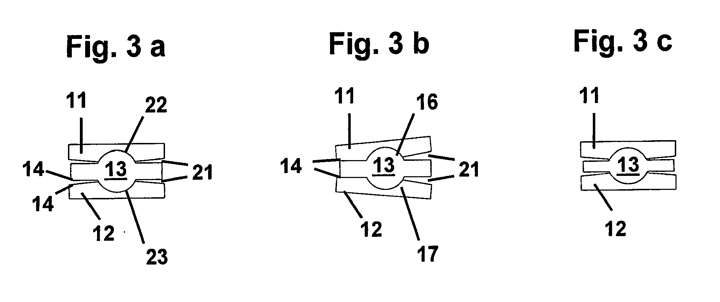

Die Figuren 3 a - c zeigen eine schematische Sagittalansicht einer dreiteiligen erfindungsgemäßen Bandscheibenendoprothese mit Rand 14 des mittleren Gleitpartners 13. Es ist jeweils der obere Gleitpartner 11, der untere Gleitpartner 12 sowie der dazwischen lokalisierte Gleitpartner 13 zu sehen. In Figur 3 a und b sind der Außenumfang der oberen Konvexität und unteren Konvexität Teil eines gemeinsamen Kreises, in Fig. 3c ist die Konvexität oben und unten und der Rand abgeflacht. In Figur 3 a ist die Prothese wieder in der Nullposition zu sehen, wobei in Figur 3 b ein dorsaler oder ventraler Lückenschluss dargestellt ist. Der Öffnungswinkel 21 hat sich in Figur 3 b jeweils entsprechend der geschlossenen Lücke auf der gegenüberliegenden Seite des konkav-konvexen Teils der Gleitflächen 22, 23 vergrößert. Es entsteht ein Lückenschluss zwischen den Rändern 14 bei endgradiger Neigung aller Gleitpartner 11, 12, 13, so dass eine optimale Druckverteilung gewährleistet ist.

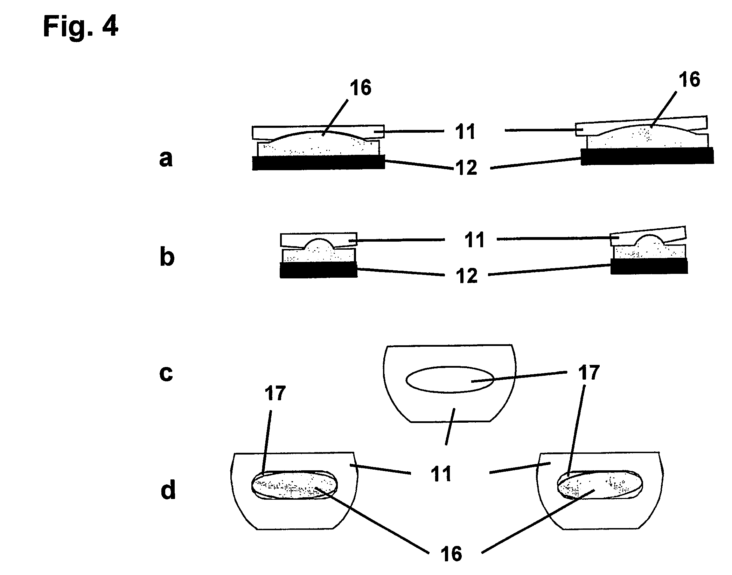

Figur 4 a - d zeigt eine schematische Darstellung einer zweiteiligen erfindungsgemäßen Bandscheibenendoprothese. Links ist jeweils die Prothese ohne Neigung und rechts mit endgradigem Lückenschluss der Gleitpartner 11, 12 dargestellt.

Figur 4 a zeigt einen medianen Frontalschnitt und in Figur 4 b ist ein medianer Sagittalschnitt durch eine erfindungsgemäße Prothese zu sehen. Der untere Gleitpartner 12 kann je nach Ausführungsform aus zwei verschiedenen Materialen bestehen, was durch die Farben grau und schwarz angedeutet ist. Dabei besteht der die Konvexität 16 aufweisende Teil (grau) aus einem anderen Material als der wirbelkörperseitige Teil des Gleitpartners 12 (schwarz). Bevorzugt sind oberer Gleitpartner 11 und unterer Gleitpartner 12 (schwarzer Teil) aus identischen Materialien. In einer Sonderform besteht nur die Konvexität 16 selbst aus einem anderen Material.

Figur 4 c zeigt eine Aufsicht auf die Innenseite eines oberen Gleitpartners 11 einer zweiteiligen Bandscheibenendoprothese. Eingezeichnet ist die konkave Ausnehmung 17, die eine zur Konvexität 16 (Figur 4 d) des unteren Gleitpartners 12 korrespondierende Form aufweist. In Figur 4 d ist ebenfalls eine Aufsicht auf die Innenseite eines oberen Gleitpartners 11 mit konkaver Ausnehmung 17 sowie Konvexität 16 des unteren Gleitpartners einer zweiteiligen Bandscheibenendoprothese dargestellt. Die konkave Ausnehmung 17 ist lateral verbreitert, wobei die Verbreiterung abgerundet ist und sich die Form der Abrundung an der Form der Konvexität orientiert. Durch die laterale Verbreiterung der Ausnehmung ist eine minimale Rotation der Konvexität 16 möglich, was im rechten Teil der Abbildung 4 d angedeutet ist.

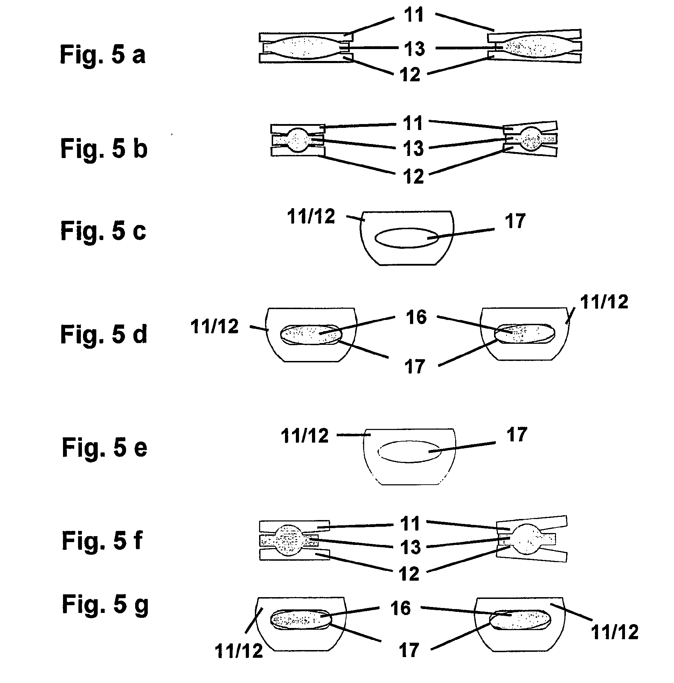

Figur 5 zeigt eine schematische Darstellung einer dreiteiligen erfindungsgemäßen Bandscheibenendoprothese mit Rand des mittleren Gleitpartners 13. In der Frontal- und Sagittalansicht ist links jeweils die Prothese ohne Neigung und rechts mit endgradigem Lückenschluss der Gleitpartner dargestellt.

In Figur 5 a ist ein medianer Frontalschnitt der Prothese dargestellt, mit oberem Gleitpartner 11, unterem Gleitpartner 12 und mittlerem Gleitpartner 13. Ein derartiger Schnitt wurde in Figur 2 bereits ausführlich beschrieben. Vorzugsweise bestehen die beiden Gleitpartner 11, 12 aus dem gleichen Material oder sind gleich beschichtet. Der mittlere Gleitpartner 13 besteht aus einem anderen Material als der obere und untere Gleitpartner oder er ist mit einer anderen Beschichtung versehen. Materialien oder Beschichtung werden vorteilhafterweise so ausgewählt, dass nach dem "low-friction-Prinzip" der Abrieb der Gleitpartner möglichst minimal ist.

In Figur 5 b ist eine dreiteilige Prothese in zentraler Sagittalansicht dargestellt. Zur ausführlicheren Beschreibung sei auf die Beschreibung zu Figur 3 a - c verwiesen, in der ebenfalls ein Sagittalschnitt einer dreiteiligen Prothese aus oberem Gleitpartner 11, unterem Gleitpartner 12 und mittlerem Gleitpartner 13, sowohl ohne Neigung der Gleitpartner 11, 12, 13 zueinander als auch mit dorsal oder ventral endgradiger Neigung dargestellt ist. Diese Ansicht ist aus Gründen der Vollständigkeit an dieser Stelle erneut abgebildet.

Figur 5c zeigt eine Transversalansicht des oberen, unteren Gleitpartners 11, 12 einer dreiteiligen Bandscheibenendoprothese mit einer Konkavität 17, die korrespondierend zur Konvexität auf Ober- und Unterseite des mittleren Gleitpartners 13 ausgestaltet ist.

Figur 5d zeigt die Transversalansicht des oberen, unteren Gleitpartners 11,12 mit lateral verbreiterter Konkavität 17, in der eine erfindungsgemäß ausgestaltete Konvexität 16 liegt (5 d links). Aufgrund der lateralen Verbreiterung der Konkavität 17 ist es möglich, dass sich die Konvexität 16 leicht in der lateral verbreiterten Ausnehmung drehen kann (5 d rechts).

In den Figuren 5e-g sind den Figuren 5b-d entsprechende Ansichten einer dreiteiligen Prothese zusehen, wobei bei diesen Prothesen (für die Lendenwirbelsäule) das Rotationszentrum nach dorsal versetzt ist.

Die Figuren 6 a - c zeigen jeweils in einer Aufsicht auf oberen und unteren Gleitpartner 11, 12 schematisch alternative Gestaltungen der Form des Außenumfanges. Dabei ist mit den kleinen Buchstaben jeweils die Orientierung hinsichtlich der dorsoventralen Ausrichtung der Platten für die Lendenwirbelsäule angegeben (d = dorsal; v = ventral), die jedoch bei der Halswirbelsäule umgekehrt ist (v dann dorsal und d dann ventral).

In den Figuren 7 a und 7 b sind für die Lendenwirbelsäule alternative Anordnungen von Verankerungszähnchen 20 auf der Außenseite des oberen und unteren Gleitpartners 11, 12 dargestellt. Auch in dieser Zeichnung ist die Orientierung der Gleitpartner hinsichtlich der dorsoventralen Ausrichtung durch die kleinen Buchstaben kenntlich gemacht (d = dorsal; v = ventral). Dorsal ist in der Mitte jeweils kein Verankerungszähnchen 20 vorgesehen, da dies einerseits eine Schonung der Wirbelkörper bewirkt und andererseits die Implantation erleichtert. Für die Halswirbelsäule gilt wieder die entgegengesetzte Orientierung, ebenfalls ohne mittleres dorsales Verankerungszähnchen 20.

Figur 8 zeigt Querschnitte durch einen mittleren Gleitpartner 13 ohne Rand und die Ableitung der Krümmungsradien aus einem Kreisumfang 19. Im oberen Teil der Abbildung haben die Konvexitäten eine gemeinsame Rotationsachse, bei der es sich um eine Sekante 18 handelt. Im Sagittalschnitt hat ein derartiger mittlerer Gleitpartner 13 einen kreisrunden Querschnitt (rechts oben). Im unteren Teil der Abbildung ist ein abgeflachter mittlerer Gleitpartner 13 dargestellt. Bei diesem fehlt mittig im Frontalschnitt ein symmetrischer Teil, angedeutet durch einen schwarzen Balken (unten mittig). Dadurch weist ein derartig aus einem Kreisumfang 19 abgeleiteter mittlerer Gleitpartner 13 im Sagittalschnitt eine eher linsenförmige Form auf (rechts unten). Im Frontalschnitt ist die Form des abgeflachten mittleren Gleitpartners 13 jedoch im Vergleich zum mittleren Gleitpartner 13, der im oberen Teil der Abbildung dargestellt ist, unverändert.

Figur 9 a - c zeigen Varianten einer erfindungsgemäßen dreiteiligen Bandscheibenendoprothese mit abgewinkeltem Rand 14 von oberem und/oder unterem Gleitpartner 11, 12 zur Sicherung des mittleren Gleitpartners 13 gegen eine Herausgleiten aus der Prothese. Durch diese erfindungsgemäße Ausgestaltung von oberem und/oder unterem Gleitpartner 11,12 wird der mittlere Gleitpartner 13 partiell oder komplett eingefasst, da dessen Rand 14 kürzer ist als die Ränder 14 von oberem und unterem Gleitpartner 11,12.

Die in den Abbildungen dargestellten Ausführungsformen der erfindungsgemäßen Bandscheibenendoprothesen sowohl in einer zweiteiligen als auch in einer dreiteiligen Ausführung sind nur beispielhaft und nicht abschließend.

### Bezugszeichenliste

- 11: oberer Gleitpartner
- 12: unterer Gleitpartner
- 13: mittlerer Gleitpartner
- 14: Rand
- 16: Konvexität
- 17: Konkavität
- 18: Sekante
- 19: Kreisumfang
- 20: Verankerungszähnchen
- 21: Öffnungswinkel
- 22: obere Artikulationsfläche
- 23: untere Artikulationsfläche

## Patentansprüche

1. Bandscheibenendoprothese, für den vollständigen Ersatz der Bandscheibe im Lenden- und Halswirbelsäulenbereich, bestehend aus artikulierenden Gleitpartnern, wobei der obere Gleitpartner Mittel für eine feste Verbindung mit einem oberen Wirbelkörper und der untere Gleitpartner Mittel für eine feste Verbindung mit einem unteren Wirbelkörper aufweist und zwischen den Gleitpartnern eine Gleitfläche angeordnet ist, wobei
a) ein erster Gleitpartner (11, 12) derart ausgebildet ist, dass die zur Verbindung mit einem Wirbelkörper entgegengesetzte Seite eine konvexe Krümmung, kwz Konvexität (16), aufweist, und
a. der Krümmungsradius der Konvexität (16)
i. in frontaler und transversaler Ansicht identisch ist und aus der Rotation des kleineren Kreisabschnittes entsteht, der sich zwischen den beiden Schnittpunkten einer Sekante (18) mit einem Kreisumfang (19) befindet, wobei die Sekante (18) nicht durch den Mittelpunkt des Kreises geht, und die Rotation um den innerhalb des Kreisumfanges (19) liegenden Abschnitt der Sekante (18) als Rotationsachse stattfindet, und
ii. in sagittaler Ansicht einem Kreisabschnitt entspricht, dessen Radius dem Abstand der Sekante (18) zu dem Kreisumfang (19) aus Unterpunkt a) a. i. entspricht, und
b. die Konvexität (16) von einem Rand (14) umschlossen wird, und
b) ein zweiter Gleitpartner (11, 12) auf der Innenseite mit einer konkaven Artikulationsfläche, kwz Konkavität (17), ausgebildet ist, und die Geometrie der Konkavität (17) **dadurch** definiert ist, dass
a. diese eine zur Konvexität (16) des ersten Gleitpartners (11, 12) korrespondierende Ausnehmung aufweist, welche
b. von einem Rand (14) umschlossen wird, und
c) die Ränder (14) beider Gleitpartner (11, 12)
a. einen sich nach außen hin öffnenden Winkel (Öffnungswinkel, 21) in Bezug zueinander aufweisen, wobei
b. sich die Öffnungswinkel (21) wenigstens im mittigen Frontalschnitt zum mittigen Sagittalschnitt durch die unterschiedliche Neigung der Ränder (14) unterscheiden, um den maximal möglichen Flächenkontakt der Ränder (14) beim endgradigen Bewegen der Gleitpartner (11, 12) zu ermöglichen, und
c. die unterschiedlichen Neigungen der Ränder (14) fließend ineinander übergehen,
d. wobei bei gleichen Öffnungswinkeln (21) in einer vertikalen Schnittebene beidseits der Artikulationsfläche die Neigungen der Ränder (14) gleich oder unterschiedlich sind, und
d) in dorsoventraler Richtung der Bewegungswinkel größer ist als in laterolateraler Richtung, welcher aus den unterschiedlichen Krümmungsradien sagittal zu frontal resultiert, und
e) die maximal mögliche Bewegung der Gleitpartner (11, 12) zueinander durch
a. Krümmungsradius und Höhe der Konvexität (16) in Bezug zum jeweiligen Rand (14), und
b. die Ausgestaltung der jeweils korrespondierenden Konkavität (17), insbesondere Höhe in Bezug zum jeweiligen Rand (14) und Form in Bezug zur korrespondierenden Konvexität (16), und
c. die schräg oder waagerecht verlaufenden umgebenden Ränder (14) der Konvexität (16) und Konkavität (17)
festgelegt wird.

2. Bandscheibenendoprothese, für den vollständigen Ersatz der Bandscheibe im Lenden- und Halswirbelsäulenbereich, bestehend aus artikulierenden Gleitpartnern, von denen der obere Gleitpartner Mittel für eine feste Verbindung mit einem oberen Wirbelkörper, und der untere Gleitpartner Mittel für eine feste Verbindung mit einem unteren Wirbelkörper aufweist, und wobei zwischen dem oberen und unteren Gleitpartner ein weiterer, mittlerer Gleitpartner angeordnet ist, der mit den Innenseiten des oberen und unteren Gleitpartners derart korrespondiert, dass eine obere und eine untere Gleitfläche entsteht, wobei
a) der mittlere Gleitpartner (13) auf Ober- und Unterseite eine konvexe Krümmung, kwz konvexität (16), aufweist, und der Krümmungsradius der Konvexität (16) auf Ober- und Unterseite
a. in frontaler und transversaler Ansicht identisch ist und aus der Rotation des kleineren Kreisabschnittes entsteht, der sich zwischen den beiden Schnittpunkten einer Sekante (18) mit einem Kreisumfang (19) befindet, wobei die Sekante (18) nicht durch den Mittelpunkt des Kreises geht, und die Rotation um den innerhalb des Kreisumfanges (19) liegenden Abschnitt der Sekante (18) als Rotationsachse stattfindet, und
b. in sagittaler Ansicht einem Kreisabschnitt entspricht, dessen Radius dem Abstand der Sekante (18) zu dem Kreisumfang aus a) a. entspricht, und
b) oberer und unterer Gleitpartner (11, 12) mit einer konkaven inneren Artikulationsfläche, kwz Konkavität (17), ausgebildet sind und die Geometrie der Konkavität (17) des oberen und unteren Gleitpartners (11, 12) jeweils **dadurch** definiert ist, dass diese jeweils eine zur Konvexität (16) der Ober- oder Unterseite des mittleren Gleitpartners (13) korrespondierende Ausnehmung aufweist, welche jeweils durch einen Rand (14) umschlossen wird, und
c) die Ränder (14) der Gleitpartner (11, 12) einen sich nach außen hin öffnenden Winkel (Öffnungswinkel, 21) in Bezug zueinander aufweisen, wobei
a. sich die Öffnungswinkel (21) wenigstens im mittigen Frontalschnitt zum mittigen Sagittalschnitt durch die unterschiedliche Neigung der Ränder (14) unterscheiden, um den maximal möglichen Flächenkontakt der Ränder (14) beim endgradigen Bewegen der Gleitpartner (11, 12, 13) zu ermöglichen, und
b. die unterschiedlichen Neigungen der Ränder (14) fließend ineinander übergehen,
c. wobei bei gleichen Öffnungswinkeln (21) in einer vertikalen Schnittebene beidseits der Artikulationsflächen die Neigungen der Ränder (14) gleich oder unterschiedlich sind, und
d) in dorsoventraler Richtung der Bewegungswinkel größer ist als in laterolateraler Richtung, welcher aus den unterschiedlichen Krümmungsradien sagittal zu frontal resultiert, und
e) die maximal mögliche Bewegung der Gleitpartner (11, 12, 13) zueinander durch
a. Krümmungsradius und Höhe der Konvexitäten (16), die Ausgestaltung der jeweils korrespondierenden Konkavitäten (17), insbesondere Höhe in Bezug zum jeweiligen Rand (14) und Form in Bezug zu korrespondierenden Konvexitäten (16), und
b. die schräg oder waagerecht verlaufenden umgebenden Ränder (14) der Konkavitäten
festgelegt wird.

3. Bandscheibenendoprothese nach Anspruch 2, wobei sich die Konvexitäten (16) des mittleren Gleitpartners (13) jeweils über die gesamte Ober- und Unterseite erstrecken oder die Konvexitäten von einem Rand (14) umgeben sind, dessen Breite gleich oder unterschiedlich ist.

4. Bandscheibenendoprothese nach wenigstens einem der Ansprüche 1 bis 3, wobei die Gleitpartner (11, 12 und 13) einstückig ausgebildet sind.

5. Bandscheibenendoprothese nach wenigstens einem der Ansprüche 1 bis 3, wobei wenigstens ein Gleitpartner (11, 12 und 13) aus wenigstens zwei fest oder fest, aber reversibel miteinander verbundenen Teilen besteht, wobei die Konvexität(en) (16) und/oder Konkavität(en) (17) den Teil ausmachen, der fest oder fest, aber reversibel mit dem jeweiligen Gleitpartner (11, 12 und 13) verbunden ist, oder Konvexität(en) (16) und/oder Konkavität(en) (17) an der Basis geeignete Mittel für eine feste oder feste, aber reversible Verbindung aufweisen.

6. Bandscheibenendoprothese nach wenigstens einem der vorhergehenden Ansprüche, wobei die Gleitpartner (11, 12 und 13) und/oder miteinander verbundene Teile aus gleichen oder verschiedenen Materialien bestehen.

7. Bandscheibenendoprothese nach wenigstens einem der vorhergehenden Ansprüche, wobei die Oberflächen der Gleitpartner (11, 12 und 13) und/oder miteinander verbundenen Teile gleich oder verschieden beschichtet sind.

8. Bandscheibenendoprothese nach wenigstens einem der Ansprüche 5 bis 7, wobei eine feste oder feste, aber reversible Verbindung durch eine Nut/Feder-Verbindung, eine Führungsschiene und korrespondierende Ausnehmung, einen Schnappmechanismus, Kleben oder Verschrauben hergestellt ist.

9. Bandscheibenendoprothese nach wenigstens einem der Ansprüche 2 bis 8, wobei oberer und unterer Gleitpartner (11, 12) aus dem gleichen Material bestehen oder gleich beschichtet sind und der mittlere Gleitpartner (13) aus einem anderen Material gefertigt ist oder anders beschichtet ist.

10. Bandscheibenendoprothese nach wenigstens einem der Ansprüche 2 bis 9, wobei die Krümmungsradien der Konvexitäten (16) auf Ober- und Unterseite des mittleren Gleitpartners (13) sowie die korrespondierenden Konkavitäten (17) auf oberem und unterem Gleitpartner (11, 12) identisch oder unterschiedlich sind.

11. Bandscheibenendoprothese nach wenigstens einem der Ansprüche 2 bis 10, wobei die maximale Höhe der Konvexitäten (16) des mittleren Gleitpartners (13) auf Ober- und Unterseite gleich oder unterschiedlich geringer ist als bei einer gemeinsamen Rotationsachse eines Kreisabschnittes nach Unterpunkt 2 a) a. und/oder bei vorhandenem Rand die Höhe des Randes (14) unverändert oder gleich oder unterschiedlich verringert ist, wobei die maximale Höhe der Konvexitäten (16) auf Ober- und Unterseite des mittleren Gleitpartners (13) gleich oder unterschiedlich ist.

12. Bandscheibenendoprothese nach wenigstens einem der vorhergehenden Ansprüche, wobei der maximale Öffnungswinkel (21) bei einseitigem Lückenschluss der Gleitpartner (11,12, und 13) aus der Extension oder Flexion zwischen 6 und 10 Grad und bei lateralem Lückenschluss zwischen 3 und 6 Grad beträgt, mit einer Toleranz von zusätzlich je 3 Grad in jede Richtung.

13. Bandscheibenendoprothese nach wenigstens einem der vorhergehenden Ansprüche, wobei die Rotation der Gleitpartner (11,12, und 13) um eine gedachte zentrale Vertikalachse bei Kongruenz der Konvexität(en) (16) und Konkavität(en) (17) zwischen den artikulierenden Gleitpartnern (11, 12, und 13) abgebremst wird.

14. Bandscheibenendoprothese nach wenigstens einem der vorhergehenden Ansprüche, wobei wenigstens eine Konkavität (17) jeweils lateral breiter ausgestaltet ist als die zugehörige Konvexität (16), wodurch eine begrenzte Rotationsbewegung der Gleitpartner (11, 12 und 13) im Bezug auf eine gedachte zentrale Vertikalachse um bis zu 3 Grad für die Lendenwirbelsäule und um bis zu 6 Grad für die Halswirbelsäule nach jeder Seite möglich ist, mit einer Toleranz von zusätzlich je 2 Grad nach jeder Seite.

15. Bandscheibenendoprothese nach wenigstens einem der vorhergehenden Ansprüche, wobei die zu einer Konvexität (16) korrespondierende Konkavität (17) als hohlkugelförmige Ausnehmung ausgebildet ist, wobei der Krümmungsradius der Konkavität (17) dem größten Krümmungsradius der zugehörigen Konvexität (16) entspricht.

16. Bandscheibenendoprothese nach wenigstens einem der vorhergehenden Ansprüche, wobei Konvexität(en) (16) und zugehörige korrespondierende Konkavität(en) (17) um bis zu 4 mm vom mittigen Sagittalschnitt nach dorsal versetzt sind.

17. Bandscheibenendoprothese nach wenigstens einem der vorhergehenden Ansprüche, wobei die Ränder (14) der Gleitpartner (11, 12 und 13) nach außen rechtwinklig, anderweitig winklig, gekrümmt oder kombiniert gerade, gekrümmt und/oder winklig abgeschlossen sind.

18. Bandscheibenendoprothese nach Anspruch 2 oder wenigstens einem darauf rückbezogenen vorhergehenden Anspruch, wobei zur zusätzlichen Sicherung eines mittleren Gleitpartners (13) mit Rand (14) gegen ein Herausgleiten aus der Prothese, beim Lückenschluss aller drei Gleitpartner (11, 12, 13) ein Anschlag Teil des äußeren Randbereichs (14) des mittleren Gleitpartners (13) ist, der außerhalb des oberen und/oder unteren Gleitpartners angeordnet ist, wobei der Anschlag wenigstens auf Ober- oder Unterseite höher ist als der Rand (14) des mittleren Gleitpartners (13) ist.

19. Bandscheibenendoprothese nach Anspruch 2 oder wenigstens einem darauf rückbezogenen vorhergehenden Anspruch, wobei zur zusätzlichen Sicherung eines mittleren Gleitpartners (13) mit Rand (14) gegen ein Herausgleiten aus der Prothese, beim Lückenschluss aller drei Gleitpartner (11, 12, 13), ein Anschlag Teil des Randes (14) des mittleren Gleitpartners (13) ist, der auf Ober- und/oder Unterseite höher als der Rand (14) des mittleren Gleitpartners (13) ist und innerhalb einer Nut im Randbereich des oberen und/oder unteren Gleitpartners (11, 12) mit dem notwendigen Spiel für die maximale Gleitbewegung der Gleitpartner (11, 12, 13) geführt wird.

20. Bandscheibenendoprothese nach Anspruch 2 oder wenigstens einem darauf rückbezogenen vorhergehenden Anspruch, wobei zur zusätzlichen Sicherung eines mittleren Gleitpartners (13) mit Rand (14) gegen ein Herausgleiten aus der Prothese, beim Lückenschluss aller drei Gleitpartner (11, 12, 13), der Rand (14) des mittleren Gleitpartners (13) ab dem Übergangsbereich der Konvexität nach peripher teilweise oder insgesamt kontinuierlich höher wird und der Rand des oberen und/oder unteren Gleitpartners (11,12) im gleichen Maße flacher wird.

21. Bandscheibenendoprothese nach Anspruch 2 oder wenigstens einem darauf rückbezogenen vorhergehenden Anspruch, wobei zur zusätzlichen Sicherung eines mittleren Gleitpartners (13) gegen ein Herausgleiten aus der Prothese, beim Lückenschluss der drei Gleitpartner (11, 12, 13) die äußeren Kanten von oberem und/oder unterem Gleitpartner (11, 12) vollständig oder partiell hakenförmig, rechtwinklig, anderweitig winklig, gekrümmt oder in Kombinationen davon in Richtung des anderen äußeren Gleitpartners ausgestaltet sind.

22. Bandscheibenendoprothese nach wenigstens einem der vorhergehenden Ansprüche, wobei Fläche und Form des Außenumfanges des oberen und unteren Gleitpartners (11, 12) gleich oder ungleich sind und so an die jeweilige Größe des Wirbelkörpers, mit dem sie verbunden werden, angepasst sind.

23. Bandscheibenendoprothese nach wenigstens einem der vorhergehenden Ansprüche, wobei oberer und/oder unterer Gleitpartner (11, 12) im Frontal- und/oder Sagittalschnitt derart ausgebildet sind, dass die Außen- und Innenseite von oberem und/oder unterem Gleitpartner (11, 12) parallel oder nicht parallel zueinander verlaufen.

24. Bandscheibenendoprothese nach wenigstens einem der vorhergehenden Ansprüche, wobei Konvexität einer zweiteiligen Prothese (11,12) und Ober- und Unterseite des mittleren Gleitpartners (13) einer dreiteiligen Prothese in Bezug zu einer Horizontalen parallel oder nicht parallel sind und dann zueinander in einem definierten Winkel stehen, wobei die Konvexität(en) symmetrisch oder asymmetrisch ist (sind).

25. Bandscheibenendoprothese nach wenigstens einem der vorhergehenden Ansprüche, wobei der obere und untere Gleitpartner (11, 12) auf der Außenseite plan oder konvex und bioaktiv beschichtet oder stumpf sind und zu deren Primärverankerung mit den Wirbelkörpern Reihen von Verankerungszähnchen (20) aufweisen, welche entweder von dorsal nach ventral seitlich gerade oder schräg angeordnet sind oder ventral und dorsal in lateraler Ausrichtung angeordnet sind, wobei sich in der dorsalen Reihe nur an den Seiten Verankerungszähnchen (20) befinden.

26. Bandscheibenendoprothese nach wenigstens einem der vorhergehenden Ansprüche, wobei der obere und/oder untere Gleitpartner (11, 12) Mittel zum Eingreifen eines Instrumentes zur Im- oder Explantation aufweisen.

27. Bandscheibenendoprothese nach wenigstens einem der vorhergehenden Ansprüche, wobei diese eine maximale Breite (Frontalansicht) von 14 bis 48 mm, eine maximale Tiefe (Sagittalschnitt) von 11 bis 35 mm und eine maximale Höhe von 4 bis 18 mm aufweist.

28. Bandscheibenendoprothese nach wenigstens einem der vorhergehenden Ansprüche geeignet für die Implantation in die Lendenwirbelsäule, wobei sich der Außenumfang des oberen und unteren Gleitpartners (11, 12) in transversaler Ansicht nach ventral verjüngt.

29. Bandscheibenendoprothese nach wenigstens einem der Ansprüche 1 bis 27 geeignet für die Implantation in die Halswirbelsäule, wobei sich der Außenumfang des oberen und unteren Gleitpartners (11, 12) in transversaler Ansicht nach dorsal verjüngt.

30. Bandscheibenendoprothese nach Anspruch 28 oder 29, wobei die Verjüngung des Außenumfangs des oberen und unteren Gleitpartners (11, 12) lateral jeweils als identische Krümmung oder asymmetrisch ausgebildet ist.

31. Bandscheibenendoprothese nach wenigstens einem der vorhergehenden Ansprüche, wobei die nicht röntgenkontrastgebenden Teile der Prothese (11, 12 und 13) jeweils unterhalb der Oberfläche ein oder mehrere röntgenkontrastgebende Markierungen enthalten.

## Claims

1. Intervertebral disc prosthesis for total replacement of the intervertebral disc within the lumbar and cervical spine, comprising articulating sliding partners, wherein the upper sliding partner comprises means for a firm assembly to an upper vertebral body and the lower sliding partner comprises means for a firm assembly to a lower vertebral body and a sliding area is located between the sliding partners, wherein
a) a first sliding partner (11, 12) is built in such a manner, that the side opposite to the assembly with a vertebral body has a convex curvature, convexity (16), and
a. the radius of curvature of the convexity (16)
i. is identical in frontal and transversal view and results from the rotation of the smaller segment of a circle, located between both intersection points of a secant (18) with a circumference (19), wherein the secant (18) does not cross the center of the circle, and the rotation axis around which the rotation takes place is the part of the secant (18) which is lying inside of the circumference (19), and
ii. corresponds in sagittal view to a segment of a circle, whose radius corresponds to the distance between the secant (18) and the circumference (19) of point a) a. i., and
b. the convexity (16) is enclosed by an edge (14), and
b) a second sliding partner (11, 12) has an inside with a concave articulation area, concavity (17), and the geometry of the concavity (17) is defined by
a. having a recess corresponding to the convexity (16) of the first sliding partner (11, 12), which
b. is enclosed by an edge (14), and
c) the edges (14) of both sliding partners (11, 12)
a. have an outwardly opening angle (aperture angle, 21) with respect to each other, wherein
b. the aperture angles (21) differ at least in the central frontal section compared to the central sagittal section by the different inclination of the edges (14), to allow the maximal possible area of contact of the edges (14) during terminal motion of the sliding partners (11, 12), and
c. the different inclinations of the edges (14) seamlessly transit,
d. wherein, at equal aperture angles (21) in a vertical section on both sides of the articulation area, the inclinations of the edges (14) are equal or different, and
d) the motion angle is greater in dorsoventral direction than in laterolateral direction resulting from the different radii of curvature sagittally to frontally, and
e) the maximal possible motion of the sliding partners (11, 12) towards each other is determined by
a. radius of curvature and height of the convexity (16) with respect to the respective edge (14), and
b. the design of each corresponding concavity (17), especially height with respect to the respective edge (14) and shape with respect to the corresponding convexity (16), and
c. the surrounding edges (14) of the convexity (16) and the concavity (17) running angular or horizontally.

2. Intervertebral disc prosthesis for total replacement of the intervertebral disc within the lumbar and cervical spine, comprising articulating sliding partners, of which the upper sliding partner comprises means for a firm assembly to an upper vertebral body and the lower sliding partner comprises means for a firm assembly to a lower vertebral body, and wherein between the upper and lower sliding partner a further, middle sliding partner is located, corresponding in such a way to the inner surfaces of the upper and lower sliding partners that an upper and a lower sliding area forms, wherein
a) the middle sliding partner (13) has on upper and lower surface a convex curvature, convexity (16), and the radius of curvature of the convexity (16) on upper and lower surface
a. is identical in frontal and transversal view and results from the rotation of the smaller segment of a circle, located between both intersection points of a secant (18) with a circumference (19), wherein the secant (18) does not cross the center of the circle, and the rotation axis around which the rotation takes place is the part of the secant (18) which is lying inside of the circumference (19), and
b. corresponds in sagittal view to a segment of a circle, whose radius corresponds to the distance between the secant (18) and the circumference of a) a., and
b) upper and lower sliding partner (11, 12) have an inside with a concave articulation area, concavity (17), and the geometry of the concavity (17) of the upper and lower sliding partner (11, 12) is each defmed by each having a recess corresponding to the convexity (16) of the upper and lower side of the middle sliding partner (13), which is each enclosed by an edge (14), and
c) the edges (14) of the sliding partners (11, 12) have an outwardly opening angle (aperture angle, 21) with respect to each other, wherein
a. the aperture angles (21) differ at least in the central frontal section compared to the central sagittal section by the different inclination of the edges (14), to allow the maximal possible area of contact of the edges (14) during terminal motion of the sliding partners (11, 12, 13), and
b. the different inclinations of the edges (14) seamlessly transit,
c. wherein, at equal aperture angles (21) in a vertical section on both sides of the articulation areas, the inclinations of the edges (14) are equal or different, and
d) the motion angle is greater in dorsoventral direction than in laterolateral direction resulting from the different radii of curvature sagittally to frontally, and
e) the maximal possible motion of the sliding partners (11, 12, 13) towards each other is determined by
a. radius of curvature and height of the convexities (16), the design of each corresponding concavities (17), especially height with respect to the respective edge (14) and shape with respect to the corresponding convexities (16), and
b. the surrounding edges (14) of the concavities running angular or horizontally.

3. Intervertebral disc prosthesis according to claim 2, wherein the convexities (16) of the middle sliding partners (13) each extend across the complete upper and lower side or the convexities are enclosed by an edge (14), whose breadth is equal or different.

4. Intervertebral disc prosthesis according to at least one of the claims 1 to 3, wherein the sliding partners (11, 12 and 13) are built as a single piece.

5. Intervertebral disc prosthesis according to at least one of the claims 1 to 3, wherein at least one sliding partner (11, 12 and 13) comprises at least two permanently or firmly, but reversibly attached parts, wherein the convexity(ies) (16) and/or the concavity(ies) (17) are the part that is permanently or firmly, but reversibly attached to the corresponding sliding partner (11, 12 and 13), or convexity(ies) (16) and/or concavity(ies) (17) have at the basis suitable means for a permanent or firm, but reversible assembly.

6. Intervertebral disc prosthesis according to at least one of the preceding claims, wherein the sliding partners (11, 12 and 13) and/or to each other attached parts comprise the same or different materials.

7. Intervertebral disc prosthesis according to at least one of the preceding claims, wherein the surfaces of the sliding partners (11, 12 and 13) and/or to each other attached parts are coated equally or differently.

8. Intervertebral disc prosthesis according to at least one of the claims 5 to 7, wherein a permanent or firm, but reversible assembly is achieved by a tongue and groove assembly, a track and corresponding recess, a snap mechanism, gluing or screwing.

9. Intervertebral disc prosthesis according to at least one of the claims 2 to 8, wherein upper and lower sliding partner (11, 12) comprise the same material or are equally coated and the middle sliding partner (13) is made of a different material or is differently coated.

10. Intervertebral disc prosthesis according to at least one of the claims 2 to 9, wherein the radii of curvature of the convexities (16) on upper and lower side of the middle sliding partner (13) as well as the corresponding concavities (17) on the upper and lower sliding partner (11, 12) are identical or different.

11. Intervertebral disc prosthesis according to at least one of the claims 2 to 10, wherein the maximal height of the convexities (16) of the middle sliding partner (13) is equally or differently less on upper and lower side than at a rotation of a segment of a circle around a common axis according to point 2 a) a. and/or at an existing edge the height of the edge (14) is unchanged or equally or differently reduced, wherein the maximal height of the convexities (16) on upper and lower side of the middle sliding partner (13) is equal or different.

12. Intervertebral disc prosthesis according to at least one of the preceding claims, wherein the maximal aperture angle (21) upon gap-closure of the sliding partners (11, 12 and 13) on one side during extension or flexion is between 6 und 10 degrees and upon lateral gap-closure between 3 und 6 degrees, with a tolerance of additionally each 3 degrees in each direction.

13. Intervertebral disc prosthesis according to at least one of the preceding claims, wherein at congruency of the convexity(ies) (16) and concavity(ies) (17) between the articulating sliding partners (11, 12 and 13) the rotation of the sliding partners (11, 12 and 13) around a fictitious central vertical axis is slowed down.

14. Intervertebral disc prosthesis according to at least one of the preceding claims, wherein at least one concavity (17) is each shaped laterally broader than the corresponding convexity (16), so that a limited rotation motion of the sliding partners (11, 12 and 13) with respect to a fictitious central vertical axis of up to 3 degrees for the lumbar spine and of up to 6 degrees for the cervical spine to each side is possible, with a tolerance of additionally each 2 degrees to each side.

15. Intervertebral disc prosthesis according to at least one of the preceding claims, wherein the concavity (17) corresponding to a convexity (16) is built as a hollow ball-shaped recess, wherein the radius of curvature of the concavity (17) matches the biggest radius of curvature of the corresponding convexity (16).

16. Intervertebral disc prosthesis according to at least one of the preceding claims, wherein convexity(ies) (16) and respective corresponding concavity(ies) (17) are dorsally displaced up to 4 mm away from the central sagittal section.

17. Intervertebral disc prosthesis according to at least one of the preceding claims, wherein the edges (14) of the sliding partners (11, 12 and 13) are terminated outwardly perpendicularly, otherwise angled, curved or a combination of straight, curved, and/or angularly.

18. Intervertebral disc prosthesis according to claim 2 or at least one preceding claim referring to it, wherein as an additional safeguard for a middle sliding partner (13) with edge (14) against a slip-out out of the prosthesis during gap-closure of all three sliding partners (11, 12, 13), a stop is part of the exterior edge area (14) of the middle sliding partner (13), which is located outside the upper and/or lower sliding partner, wherein the stop on at least its upper or lower side is higher than the edge (14) of the middle sliding partner (13).

19. Intervertebral disc prosthesis according to claim 2 or at least one preceding claim referring to it, wherein as an additional safeguard for a middle sliding partner (13) with edge (14) against a slip-out out of the prosthesis during gap-closure of all three sliding partners (11, 12, 13), a stop is part of the edge (14) of the middle sliding partner (13), which is higher on its upper and/or lower side than the edge (14) of the middle sliding partner (13) and is guided within a groove in the edge area of the upper and/or lower sliding partner (11, 12) with the clearance necessary for the maximal sliding motion of the sliding partners (11, 12, 13).

20. Intervertebral disc prosthesis according to claim 2 or at least one preceding claim referring to it, wherein as an additional safeguard for a middle sliding partner (13) with edge (14) against a slip-out out of the prosthesis during gap-closure of all three sliding partners (11, 12, 13) the edge (14) of the middle sliding partner (13) increases continuously partly or totally in height from the transition area of the convexity to a periphery and the edge of the upper and/or lower sliding partner (11, 12) levels off to the same degree.

21. Intervertebral disc prosthesis according to claim 2 or at least one preceding claim referring to it, wherein as an additional safeguard for a middle sliding partner (13) against a slip-out out of the prosthesis during gap-closure of the three sliding partners (11, 12, 13), the exterior borders of the upper and/or lower sliding partner (11, 12) are completely or partially hook-shaped, perpendicular, otherwise angular, curved or a combination thereof in direction of the other exterior sliding partner.

22. Intervertebral disc prosthesis according to at least one of the preceding claims, wherein surface and shape of the outer circumference of the upper and lower sliding partner (11, 12) are equal or different and are thereby adapted to the corresponding size of the vertebral body with which they are assembled.

23. Intervertebral disc prosthesis according to at least one of the preceding claims, wherein upper and/or lower sliding partner (11, 12) are designed in such a way in the frontal and/or sagittal section that the outside and inside of the upper and/or lower sliding partner (11, 12) run in parallel or non-parallel relative to one another.

24. Intervertebral disc prosthesis according to at least one of the preceding claims, wherein convexity of a two-part prosthesis (11, 12) and upper and lower side of the middle sliding partner (13) of a three-part prosthesis are parallel or non-parallel with respect to a horizontal, and thus are in a defined angle relative to each other, wherein the convexity(ies) is (are) symmetrical or asymmetrical.

25. Intervertebral disc prosthesis according to at least one of the preceding claims, wherein the upper and lower sliding partner (11, 12) are on the outer surface plane or convex and coated bio-actively or are blunt and have for their primary anchorage with the vertebral bodies rows of anchoring teeth (20), that are either arranged from dorsal to ventral laterally straight or angular or ventrally and dorsally in lateral alignment, wherein the dorsal row has only laterally arranged anchoring teeth (20).

26. Intervertebral disc prosthesis according to at least one of the preceding claims, wherein the upper and/or lower sliding partner (11, 12) have means for an instrument to grip for implantation or explantation.

27. Intervertebral disc prosthesis according to at least one of the preceding claims, wherein it has a maximal breadth (frontal view) of 14 to 48 mm, a maximal depth (sagittal section) of 11 to 35 mm and a maximal height of 4 to 18 mm.

28. Intervertebral disc prosthesis according to at least one of the preceding claims, suitable for implantation into the lumbar spine, wherein the outer circumference of the upper and lower sliding partner (11, 12) tapers off ventrally in the transversal view.

29. Intervertebral disc prosthesis according to at least one of the claims 1 to 27, suitable for implantation into the cervical spine, wherein the outer circumference of the upper and lower sliding partner (11, 12) tapers off dorsally in the transversal view.

30. Intervertebral disc prosthesis according to claim 28 or 29, wherein the tapering off of the outer circumference of the upper and lower sliding partner (11, 12) has laterally identical curvature or is asymmetric.

31. Intervertebral disc prosthesis according to at least one of the preceding claims, wherein the non radio-opaque parts of the prosthesis (11, 12 and 13) each comprise under the surface one or more radio-opaque marks.

## Revendications

1. Endoprothèse de disque pour le remplacement total du disque dans la zone de la colonne lombaire et de la colonne cervicale, comprenant des paliers de glissement d'articulation, le palier de glissement supérieur comportant un moyen pour une liaison fixe avec un corps vertébral supérieur et le palier de glissement inférieur comportant un moyen pour une liaison fixe avec un corps vertébral inférieur et une surface de glissement étant disposée entre les paliers de glissement, dans laquelle
a) un premier palier de glissement (11,12) est conçu de manière que le côté opposé à la liaison au corps vertébral comporte une courbure convexe (16), convexité en abrégé, et
a. le rayon de courbure de la convexité (16)
i. est identique en vue frontale et transversale et résulte de la rotation de la section de cercle plus petite, qui se trouve entre les deux points de coupe d'une sécante (18) avec une périphérie (19), la sécante (18) ne traversant pas le point central du cercle, et la rotation ayant lieu autour de la section se trouvant à l'intérieur de la périphérie (19) de la sécante (18) comme axe de rotation, et
ii. correspond en vue sagittale à une section de cercle, dont le rayon correspond à l'écart de la sécante (18) par rapport à la périphérie (19) du sous-point a)a. i., et
b. la convexité (16) est fermée par une bordure (14), et
b) un deuxième palier de glissement (11,12) est conçu sur le côté intérieur avec une surface d'articulation concave, concavité en abrégé (17), et la géométrie de la concavité (17) est définie en ce que
a. celle-ci comporte un évidement correspondant à la convexité (16) du premier palier de glissement (11,12), qui
b. est fermé par une bordure (14) et
c) les bordures (14) des deux paliers de glissement (11,12)
a. comportent un angle ouvrant (angle d'ouverture, 21) vers l'extérieur l'un par rapport à l'autre,
b. les angles d'ouverture (21) se différenciant au moins dans la section frontale centrale par rapport à la section sagittale par une inclinaison différente des bordures (14) afin de permettre le contact de surface maximal possible les bordures (14) lors d'un mouvement en position terminale des paliers de glissement (11,12) et
c. les différentes inclinaisons des bordures (14) passent de manière fluide les unes dans les autres,
d. les inclinaisons des bordures (14) étant identiques ou différentes pour des angles d'ouverture (21) identiques dans un niveau de coupe vertical des deux côtés de la surface d'articulation, et
d) l'angle de mouvement est supérieur dans le sens dorsoventral à celui du sens latéro-latéral, lequel résulte des différents rayons de courbure sagittal par rapport à frontal, et
e) le mouvement maximal possible des paliers de glissement (11,12) l'un par rapport à l'autre est déterminé par
a. le rayon de courbure et la hauteur de la convexité (16) par rapport à la bordure correspondante (14), et
b. la conception de la concavité correspondante (17), en particulier la hauteur par rapport à la bordure correspondante (14) et la forme par rapport à la convexité correspondante (16), et
c. les bordures (14) entourantes disposées de manière inclinée ou horizontale de la convexité (16) et de la concavité (17).

2. Endoprothèse de disque pour le remplacement total du disque dans la zone de la colonne lombaire et de la colonne cervicale, comprenant des paliers de glissement d'articulation, dans lesquels le palier de glissement supérieur comporte un moyen pour une liaison solide avec un corps vertébral supérieur et le palier de glissement inférieur comporte un moyen pour une liaison solide avec un corps vertébral inférieur et une surface de glissement étant disposée entre les paliers de glissement, et dans laquelle un autre palier de glissement central est disposé entre le palier de glissement supérieur et le palier de glissement inférieur, lequel correspond avec les côtés intérieurs des paliers de glissement supérieur et inférieur de sorte qu'une surface de glissement supérieure et une surface de glissement inférieure est créée, dans laquelle
a) le palier de glissement central (13) comporte sur le côté supérieur et le côté inférieur une courbure convexe (16), convexité en abrégé, et le rayon de courbure de la convexité (16) sur le côté supérieur et inférieur
a. est identique en vue frontale et transversale et résulte de la rotation de la section de cercle plus petite, qui se trouve entre les deux points de coupe d'une sécante (18) avec une périphérie (19), la sécante (18) ne traversant pas le point central du cercle, et la rotation ayant lieu autour de la section se trouvant à l'intérieur de la périphérie (19) de la sécante (18) comme axe de rotation, et
b. correspond en vue sagittale à une section de cercle, dont le rayon correspond à l'écart de la sécante (18) par rapport à la périphérie du sous-point a) a., et
b) les paliers de glissement (11,12) supérieur et inférieur sont conçus avec une surface d'articulation interne concave (17), concavité en abrégé, et la géométrie de la concavité (17) du palier de glissement supérieur et inférieur (11,12) est définie, respectivement, de manière à comporter un évidement correspondant à la convexité (16) du côté supérieur et inférieur du palier de glissement central (13), qui est fermée par une bordure (14,) et
c) les bordures (14) des paliers de glissement (11,12) comportent un angle ouvrant (angle d'ouverture, 21) vers l'extérieur l'un par rapport à l'autre,
a. les angles d'ouverture (21) se différenciant au moins dans la section frontale centrale par rapport à la section sagittale par une inclinaison différente des bordures (14) afin de permettre le contact de surface maximal possible des bordures (14) lors d'un mouvement en position terminale des paliers de glissement (11,12,13) et
b. les différentes inclinaisons des bordures (14) passant de manière fluide les unes dans les autres,
c. les inclinaisons des bordures (14) étant identiques ou différentes pour des angles d'ouverture (21) identiques dans un niveau de coupe vertical des deux côtés de la surface d'articulation, et
d) l'angle de mouvement étant supérieur dans le sens dorsoventral à celui du sens latérolatéral, lequel résulte des différents rayons de courbure sagittal par rapport à frontal, et
e) le mouvement maximal possible des paliers de glissement (11,12,13) les uns par rapport aux autres étant déterminé par
a. le rayon de courbure et la hauteur des convexités (16), la conception de la concavité correspondante (17), en particulier la hauteur par rapport à la bordure correspondante (14) et la forme par rapport à la convexité correspondante (16), et
b. les bordures (14) entourantes disposées de manière inclinée ou horizontale des concavités.

3. Endoprothèse de disque selon la revendication 2, dans laquelle les convexités (16) du palier de glissement central (13) s'étendent respectivement sur tout le côté supérieur et inférieur ou les convexités sont entourées par une bordure (14), dont la largeur est identique ou différente.

4. Endoprothèse de disque selon au moins l'une des revendications 1 à 3, dans laquelle les paliers de glissement (11,12 et 13) sont conçus en une seule pièce.

5. Endoprothèse de disque selon au moins l'une des revendications 1 à 3, dans laquelle au moins un palier de glissement (11,12 et 13) comprend au moins deux parties reliées l'une à l'autre fixement ou fixement, mais de manière réversible, la/les convexité(s) (16) et/ou concavité(s) (17) formant la pièce, qui est reliée fixement ou fixement, mais de manière réversible, au palier de glissement correspondant (11,12 et 13), ou la/les convexité(s) (16) et/ou concavités() (17) comportent à la base des moyens adaptés à une liaison fixe ou fixe, mais réversible.

6. Endoprothèse selon au moins l'une des revendications précédentes, dans laquelle les paliers de glissement (11,12 et 13) et/ou les parties reliées les unes aux autres comprennent des matériaux identiques ou différents.

7. Endoprothèse de disque selon au moins l'une des revendications précédentes, dans laquelle les surfaces des paliers de glissement (11,12 et 13) et/ou des pièces reliées les unes aux autres sont recouvertes de manière identique ou différente.

8. Endoprothèse de disque selon au moins l'une des revendications 5 à 7, dans laquelle une liaison fixe ou fixe, mais réversible, est générée par une liaison rainure/ressort, un rail de guidage et un évidement correspondant, un mécanisme de cliquet, un collage ou un vissage.

9. Endoprothèse de disque selon au moins l'une des revendications 2 à 8, dans laquelle les paliers de glissement supérieur et inférieur (11,12) comprennent le même matériau ou sont recouverts de manière identique et le palier de glissement central (13) est fabriqué dans un autre matériau ou est recouvert de manière différente.

10. Endoprothèse de disque selon au moins l'une des revendications 2 à 9, dans laquelle les rayons de courbure des convexités (16) sur le côté supérieur et inférieur du palier de glissement central (13) ainsi que les convexités correspondantes (17) sur les paliers de glissement supérieur et inférieur (11,12) sont identiques ou différents.

11. Endoprothèse de disque selon l'une des revendications 2 à 20, dans laquelle la hauteur maximale des convexités (16) du palier de glissement central (13) est plus petit de manière identique ou différente sur le côté supérieur et inférieur que dans un axe de rotation commun d'une section de cercle selon le point 2 a) a. et/ou au niveau d'une bordure existante la hauteur de la bordure (14) est inchangée ou réduite de manière identique ou différente, la hauteur maximale des convexités (16) sur le côté supérieur et inférieur du palier de glissement central (13) étant identique ou différente.

12. Endoprothèse de disque selon au moins l'une des revendications précédentes, dans laquelle l'angle d'ouverture maximal (21) s'élève entre 3 et 6 degrés pour une fermeture d'espace des paliers de glissement (11,12 et 13) d'un côté pendant l'extension ou la flexion, et entre 3 et 6 degrés pour la fermeture d'espace latéral, avec une tolérance de 3 degrés supplémentaires dans chaque direction.

13. Endoprothèse de disque selon au moins l'une des revendications précédentes, dans laquelle la rotation des paliers de glissement (11,12 et 13) est freinée autour d'un axe vertical central virtuel lors de la congruence de la/des convexité(s) (16) et de la/des concavité(s) (17) entre les paliers de glissement d'articulation (11,12 et 13).

14. Endoprothèse de disque selon au moins l'une des revendications précédentes, dans laquelle au moins une concavité (17) est conçue respectivement plus large latéralement que la convexité correspondante (16), un mouvement de rotation limité des paliers de glissement (11,12 et 13) par rapport à un axe vertical central virtuel étant ainsi possible jusqu'à 3 degrés pour la colonne vertébrale et jusqu'à 6 degrés pour la colonne cervicale dans chaque côté, avec une tolérance de 2 degrés supplémentaires dans chaque côté.

15. Endoprothèse de disque selon au moins l'une des revendications précédentes, dans laquelle la concavité (17) correspondant à une convexité (16) est conçue comme un évidement en forme de bille creuse, le rayon de courbure de la convexité (17) correspondant au plus grand rayon de courbure de la convexité (16) correspondante.

16. Endoprothèse de disque selon au moins l'une des revendications précédentes, dans laquelle la/les convexités (16) et la/les concavités correspondantes (17) sont décalées vers la section dorsale de jusqu'à 4 mm par rapport à la section sagittale centrale.

17. Endoprothèse de disque selon au moins l'une des revendications précédentes, dans laquelle les bordures (14) des paliers de glissement (11,12 et 13) sont fermées rectangulairement vers l'extérieur, angulairement, de manière courbée ou droite associée, de manière courbée et/ou angulaire.

18. Endoprothèse de disque selon la revendication 2 ou au moins l'une des revendications précédente s'y rapportant, dans laquelle, en tant que sécurité supplémentaire pour un palier de glissement central (13) avec bordure (14) contre les sorties hors de la prothèse lors de la fermeture d'espace des trois paliers de glissement (11,12,13), une butée fait partie de la zone de bordure externe (14) du palier de glissement central (13), laquelle est disposée en dehors du palier de glissement supérieur et/ou inférieur, la butée étant plus haute au moins sur le côté supérieur ou inférieur que la bordure (14) du palier de glissement central (13).

19. Endoprothèse selon la revendication 2 ou au moins l'une des revendications précédentes s'y rapportant, dans laquelle, en tant que sécurité supplémentaire pour un palier de glissement central (13) avec bordure (14) contre la sortie en dehors de la prothèse lors de la fermeture d'espace des trois paliers de glissement (11,12,13), une butée fait partie de la bordure (14) du palier de glissement central (13), laquelle est plus haute sur le côté supérieur et/ou inférieur que la bordure (14) du palier de glissement central (13) et est insérée à l'intérieur d'une rainure dans la zone de bordure du palier de glissement supérieur et/ou inférieur (11,12) avec le jeu nécessaire pour le mouvement de glissement maximal des paliers de glissement (11,12,13).

20. Endoprothèse de disque selon la revendication 2 ou au moins l'une des revendications précédentes s'y rapportant, dans laquelle, en tant que sécurité supplémentaire pour un palier de glissement central (13) avec bordure (14) contre la sortie en dehors de la prothèse lors de la fermeture d'espace des trois paliers de glissement (11,12,13), la bordure (14) du palier de glissement central (13) devient plus haute à partir de la zone de passage de la convexité partiellement vers la périphérie, partiellement ou globalement en continu et la bordure du palier de glissement supérieur et/ou inférieur (11,12) est plus plat dans une mesure identique.

21. Endoprothèse de disque selon la revendication 2 ou au moins l'une des revendications précédentes s'y rapportant, dans laquelle en tant que sécurité supplémentaire pour un palier de glissement central (13) contre la sortie en dehors de la prothèse lors de la fermeture d'espace des trois paliers de glissement (11,12,13), les bordures externes du palier de glissement supérieur et/ou inférieur (11,12) sont conçues totalement ou partiellement en forme de crochet, rectangulairement, angulairement autrement, de manière courbée ou en association de cela en direction des autres paliers de glissement externes.

22. Endoprothèse de disque selon au moins l'une des revendications précédentes, dans laquelle les surfaces ou la forme de la périphérie extérieure du palier de glissement supérieur et inférieur (11,12) sont identiques ou différentes et sont ainsi adaptées aux dimensions correspondantes du corps vertébral, auquel elles sont reliées.

23. Endoprothèse de disque selon au moins l'une des revendications précédentes, dans laquelle les paliers de glissement supérieur et/ou inférieur (11,12) sont conçus en coupe frontale et/ou sagittale de sorte que les côtés extérieur et intérieur des paliers de glissement supérieur et/ou inférieur (11,12) sont disposés de manière parallèle ou non parallèle les uns aux autres.

24. Endoprothèse de disque selon au moins l'une des revendications précédentes, dans laquelle la convexité d'une prothèse en deux parties (11,12) et les côtés supérieur et inférieur du palier de glissement central (13) d'une prothèse en trois parties sont parallèles ou non parallèles par rapport à une horizontale et se trouvent ainsi dans un angle défini l'un par rapport à l'autre, la/les convexité(s) étant symétrique(s) ou asymétrique(s).

25. Endoprothèse de disque selon au moins l'une des revendications précédentes, dans laquelle les paliers de glissement supérieur et inférieur (11,12) sont recouverts sur le côté externe de manière plan ou convexe et bioactive ou sont émoussés et comportent des rangées de petites dents d'ancrage (20) pour son ancrage primaire avec les corps vertébraux, lesquelles sont disposées de manière droite ou inclinée latéralement du dos vers le ventre et dans le sens latéral ventralement et dorsalement, des rangées se trouvant uniquement sur les côtés des petites dents d'ancrage (20) dans le sens dorsal.

26. Endoprothèse de disque selon au moins l'une des revendications précédentes, dans laquelle les paliers de glissement supérieur et/ou inférieur (11,12) comportent des moyens de mise en prise des instruments d'implantation ou d'explantation.

27. Endoprothèse de disque selon au moins l'une des revendications précédentes, dans laquelle celle-ci comporte une largeur maximale (vue de face) de 14 à 48 mm, une profondeur maximale (coupe sagittale) de 11 à 35 mm et une hauteur maximale de 4 à 18 mm.

28. Endoprothèse de disque selon au moins l'une des revendications précédentes adaptée à une implantation dans la colonne vertébrale, dans laquelle la périphérie externe des paliers de glissement supérieur et inférieur (11,12) se réduit en vue transversale vers le ventre.

29. Endoprothèse de disque selon au moins l'une des revendications 1 à 27 adaptée à une implantation dans la colonne vertébrale, dans laquelle la périphérie externe des paliers de glissement supérieur et inférieur (11,12) se réduit en vue transversale vers le dos.

30. Endoprothèse de disque selon la revendication 28 ou 29, dans laquelle le rétrécissement de la périphérie extérieure des paliers de glissement supérieur et inférieur (11,12) est conçu latéralement comme une courbure identique ou de manière asymétrique.

31. Endoprothèse de disque selon au moins l'une des revendications précédentes, dans laquelle les parties de la prothèse (11,12 et 13) non visibles aux rayons X contiennent respectivement sous la surface un ou plusieurs marquages visibles aux rayons X.
